# EUROPÄISCHE PATENTSCHRIFT

(11) **EP 3 496 699 B1**
(45) Veröffentlichungstag und Bekanntmachung des Hinweises auf die Patenterteilung: **13.05.2020**
(21) Anmeldenummer: 17751698.6
(22) Anmeldetag: 03.08.2017
(51) Int. Cl.: A61K 8/42, A61K 8/60, A61Q 19/00, A61Q 5/02, A61Q 19/10

(54) **N-METHYL-N-((2S,3R,4R,5R)-2,3,4,5,6-PENTAHYDROXYHEXYL)-(Z)-OCTADEC-9-ENAMID ZUR VERRINGERUNG DER AUGENREIZUNG**
N-METHYL-N-((2S,3R,4R,5R)-2,3,4,5,6-PENTAHYDROXYHEXYL)-(Z)-OCTADEC-9-ENAMID FOR ALLEVIATING EYE IRRITATION
N-METHYL-N-((2S,3R,4R,5R)-2,3,4,5,6-PENTAHYDROXYHEXYL)-(Z)-OCTADEC-9-ENAMIDE POUR ATTÉNUER L'IRRITATION DES YEUX

(30) Priorität: 08.08.2016 DE 102016114673
(43) Veröffentlichungstag der Anmeldung: 19.06.2019
(73) Patentinhaber: Werner & Mertz GmbH, 55120 Mainz (DE)
(72) Erfinder: MÜLLERHEIM, Jennifer, 55411 Bingen (DE); ENDLEIN, Edgar, 29559 Wrestedt (DE)
(74) Vertreter: Eisenführ Speiser
(86) Internationale Anmeldenummer: PCT/EP2017/069642
(87) Internationale Veröffentlichungsnummer: WO 2018/029069

(56) Entgegenhaltungen:
- EP-A1- 0 285 768
- WO-A2-2014/206555

## Beschreibung

Der Gegenstand der vorliegenden Erfindung wird in den Ansprüchen definiert.

Offenbart werden eine Zusammensetzung, umfassend ein, zwei oder mehr Tenside ausgewählt aus der Gruppe bestehend aus Sodium Laureth Sulfate, Cocoamidopropyl Betain und Sodium Laurylsulfate sowie zum Abmildern oder Vermeiden von Augenreizungen N-Methyl-N-((2S,3R,4R,5R)-2,3,4,5,6-pentahydroxyhexyl)-(Z)-octadec-9-enamid sowie ein, zwei oder mehr weitere N-Methyl-N-((2S,3R,4R,5R)-2,3,4,5,6-pentahydroxyhexyl)-Fettsäureamide, die Verwendung der offenbarten Zusammensetzung als Spülmittel oder Reinigungsmittel, die Verwendung der offenbarten Zusammensetzung als Additiv zur Verringerung der augenreizenden Wirkung von Spülmitteln oder Reinigungsmitteln, die Verwendung der offenbarten Zusammensetzung als Mittel zur Verringerung der augenreizenden Wirkung bestimmter Tenside, entsprechende Verfahren zur Herstellung eines Spülmittels sowie entsprechende Verfahren zur Herstellung der offenbarten Zusammensetzung.

Spülmittel und Reinigungsmittel umfassen als Aktivstoffe insbesondere Tenside. Diese Tenside sind verantwortlich für die Leistungskraft der Spül- und Reinigungsmittel. Sie besitzen über ihre Reinigungswirkung hinaus jedoch auch eine Vielzahl weiterer Eigenschaften, die bei der Konzeption moderner Spül- und Reinigungsmittel zu berücksichtigen sind. Tenside, deren Einsatz in einem Spül- oder Reinigungsmittel eine hervorragende Leistungskraft (d.h. eine besondere Spül- oder Reinigungsleistung) bewirkt, können zusätzliche positive oder negative Eigenschaften besitzen.

Als negative Eigenschaft eines Spül- oder Reinigungsmittels und der darin enthaltenen Tenside wird es regelmäßig angesehen, wenn diese Spül- beziehungsweise Reinigungsmittel bei vorgesehener Benutzung die Haut und/oder die Augen reizen.

Als nachteilig wird es auch empfunden, wenn ein bestimmtes Tensid in der Natur oder in Kläranlagen nur mangelhaft abgebaut werden kann.

Ebenfalls als nachteilig wird es empfunden, wenn ein bestimmtes Tensid aus dem Rohstoff Erdöl synthetisiert wird, weil dieser Rohstoff nicht in den natürlichen CO₂-Kreislauf eingebunden ist, sodass seine Nutzung zur Anreicherung des klimaschädlichen CO₂ beiträgt.

Zunehmend bevorzugt sind deshalb im unmittelbaren Vergleich Tenside auf Basis regenerativer Pflanzenöle (sogenannte native Tenside); diese Tenside entstehen durch Synthese aus nachhaltigen und erneuerbaren Rohstoffquellen, die Teil des natürlichen CO₂-Kreislaufs sind. Typische Beispiele für entsprechende Tenside sind Fettalkoholethersulfate und Fettalkoholsulfate als Vertreter von anionischen Tensiden und Fettalkoholethoxylate als nichtionische Tenside. Weil der Fettalkoholanteil solcher Tenside naturbedingt linear und geradzahlig ist sowie eine primäre Alkoholfunktionalität aufweist, eignet er sich hervorragend für die biologische Abbaubarkeit, im Vergleich mit erdölbasierten Tensiden.

Die hydrophilen funktionellen Gruppen der vorstehend genannten Tenside auf Basis regenerativer Pflanzenöle sind regelmäßig in terminaler Position angeordnet, was sich positiv auf die Reinigungskraft auswirkt. Aus diesem und anderen Gründen werden die genannten Tenside auf Basis regenerativer Pflanzenöle (insbesondere Fettalkoholethersulfate, Fettalkoholsulfate und Fettalkoholethoxylate) in einer Vielzahl von Spül- und Reinigungsmitteln, insbesondere auch Handgeschirrspülmitteln, eingesetzt.

Als nachteilig wird jedoch zunehmend empfunden, dass die vorstehend genannten Tenside auf Basis regenerativer Pflanzenöle regelmäßig auf den Pflanzenölen Palm- bzw. Palmkernöl und Kokosöl basieren, deren Anbau in Ländern, wie zum Beispiel Indonesien und Malaysia, mit einem verstärkten Raubbau am tropischen Regenwald einhergeht. Zudem wird es als nachteilig empfunden, dass die genannten nativen Tenside zwar durchaus leistungsstark sind, sie jedoch in Einzelfällen verantwortlich gemacht werden für bei Benutzung entsprechender Spül- oder Reinigungsmittel auftretende Haut- und/oder Augenreizungen.

Spül- und Reinigungsmittel basierend auf den vorstehend genannten leistungsstarken, d.h. meist stark entfettenden Tensiden, können haut und augenreizend sein.

Es wurde bereits versucht, potenzielle Reizwirkungen z.B. auf die menschliche Haut durch Einsatz sogenannter Co-Tenside abzumildern. Beispiele für zu diesem Zweck eingesetzte Co-Tenside sind Cocamidopropyl Betaine (CAPB), Alkylbetaine und Alkylaminoxide als Vertreter zwitterionischer Molekülstrukturen. Zu dem gleichen Zweck eingesetzte anionische Tenside sind zum Beispiel Glutamate, Sarcosinate, Taurate, Sulfosuccinate, Ether Carboxylate und andere. Als nichtionische Co-Tenside wurden für den gleichen Zweck vor allem Alkylpolyglycoside (APG) eingesetzt. Alkylpolyglycoside gehören zur Gruppe der Zuckertenside und stellen ihren wichtigsten Vertreter dar. Eine weitere wichtige Gruppe der Zuckertenside sind N-Methylglucamide, welche durch reduktive Aminierung von Glucose in Gegenwart von Methylamin und anschließende Acylierung des so erhaltenen N-Methylglucamins mit Fettsäurederivaten hergestellt werden. N-Methylglucamide werden gemäß IUPAC als N-Methyl-N-((2S,3R,4R,5R)-2,3,4,5,6-pentahydroxyhexyl)-Fettsäureamide benannt, vereinzelt jedoch auch als 1-Deoxy-1-(methyl-(alkanoyl)amino)-D-Glucitole bezeichnet. N-Methylglucamide sind zwar als hautmild, gut biologisch abbaubar und hinsichtlich ihrer oberflächenaktiven Eigenschaften als mit APG vergleichbar bezeichnet worden. Die bislang untersuchten M-Methylglucamide sind nachteiligerweise jedoch noch immer augenschädigend (vgl. beispielsweise die Sicherheitsdatenblätter gemäß Verordnung (EU) Nr. 453/2010) zu den Handelsprodukten GlucoPure Wet und GlucoPure Deg der Firma Clariant, welche N-Methylglucamide mit C8-C10 bzw. C12-C14 Fettsäureresten umfassen). Die genannten Co-Tenside sind somit zwar im Einzelfall durchaus in der Lage, in Kombination mit (Haupt-)Tensiden die Rezepturen von Spül- und Reinigungsmitteln (insbesondere Handgeschirrspülmitteln) abzumildern und insbesondere hautmilder zu gestalten. Nachteiligerweise sind die resultierenden marktgängigen Rezepturen jedoch trotz der Gegenwart der besagten Co-Tenside noch immer augenreizend.

Zusammensetzungen, insbesondere Spülmittelzusammensetzungen, umfassend ein, zwei oder mehr Tenside ausgewählt aus der Gruppe bestehend aus Sodium Laureth Sulfate, Cocoamidopropyl Betain und Sodium Laurylsulfate sind bereits bekannt und werden in der Praxis aufgrund ihrer hervorragenden Reinigungsleistung vielfach eingesetzt. Es besteht jedoch ein Bedarf an besonders augenmilden Zusammensetzungen umfassend ein, zwei oder mehr dieser Tenside.

Die WO 2014/206554 A2 betrifft die Verwendung von speziellen N-Alkyl-N-acylglucaminen in Hautreinigungsmitteln sowie Hautreinigungsmittel enthaltend diese N-Alkyl-N-acylglucamine. Die WO 2013/178697 A2 betrifft die Verwendung von N-Methyl-N-acylglucaminen als Verdicker in Tensidlösungen sowie kosmetische Zusammensetzungen, enthaltend solche Tensidlösungen. Die vorliegende Erfindung basiert auf der primären Aufgabe, eine Zusammensetzung umfassend ein, zwei oder mehr (d.h. sämtliche) Tenside ausgewählt aus der Gruppe bestehend aus Sodium Laureth Sulfate, Cocoamidopropyl Betain und Sodium Laurylsulfate anzugeben, welche eine hohe Reinigungsleistung besitzt, dabei aber selbst in besonderem Maße augenmild ist.

Vorzugsweise sollte die anzugebende Zusammensetzung neben den bereits genannten Tensiden zumindest einen weiteren Aktivstoff enthalten, der für die Augenmilde verantwortlich ist. Mit anderen Worten sollte vorzugsweise wegen dessen Anwesenheit die Zusammensetzung augenmilder sein, als es bei dessen Abwesenheit der Fall wäre. Vorzugsweise sollte ein solcher die Augenmilde verursachender Aktivstoff selbst ein Tensid sein. Somit sollte die anzugebende Zusammensetzung neben den bereits genannten Tensiden vorzugsweise zumindest ein Tensid umfassen, das nicht nur selbst augenmild ist, sondern auch dazu befähigt ist, die augenreizende bzw. augenschädigende Wirkung von insbesondere den bereits genannten Tensiden (vorzugsweise aber auch anderen üblichen Bestandteilen von Spül- bzw. Reinigungsmitteln) abzumildern, und zwar vorteilhafterweise nicht lediglich durch bloße Verdünnung der augenreizenden Aktivstoffe, sondern im Sinne einer echten (synergistischen) Wechselwirkung mit diesen.

Dieses vorzugsweise einzusetzende, die Augenmilde bewirkende Tensid wiederum sollte vorzugsweise nicht erdölbasiert sein, sondern unter Verwendung nachhaltiger, erneuerbarer Rohstoffquellen (auf Pflanzenbasis) synthetisiert werden können, wobei vorzugsweise weder Palm- bzw. Palmkernöl noch Kokosöl die Basis für das Tensid sein sollte.

Vorteilhafterweise sollte die anzugebende Zusammensetzung in der Natur und in Kläranlagen leicht abgebaut werden können; sofern die anzugebende Zusammensetzung einen zusätzlichen Aktivstoff enthält, sollte zumindest dieser Aktivstoff in der Natur und in Kläranlagen leicht abgebaut werden können.

Besonders vorzugweise sollte die anzugebende Zusammensetzung neben den bereits genannten Tensiden ein, zwei oder mehr weitere Tenside zum Abmildern oder Vermeiden von Augenreizungen umfassen.

Die vorliegende Erfindung basiert auf der weiteren Aufgabe, eine Verwendung der anzugebenden Zusammensetzung als Spülmittel oder als Reinigungsmittel vorzusehen. Darüber hinaus sollte vorzugsweise die Verwendung der anzugebenden Zusammensetzung als Additiv zur Verringerung der augenreizenden Wirkung von Spülmitteln bzw. von Reinigungsmitteln angegeben werden.

Entsprechend war es eine Aufgabe der vorliegenden Erfindung, einen Aktivstoff bzw. eine Mischung von Aktivstoffen anzugeben zur Verwendung
- als Additiv zur Verringerung der augenreizenden Wirkung von Spülmitteln umfassend ein, zwei oder mehr Tenside ausgewählt aus der Gruppe bestehend aus Sodium Laureth Sulfate, Cocoamidopropyl Betain und Sodium Laurylsulfate oder von Reinigungsmitteln umfassend ein, zwei oder mehr Tenside ausgewählt aus der Gruppe bestehend aus Sodium Laureth Sulfate, Cocoamidopropyl Betain und Sodium Laurylsulfate, für ein oder mehrere Substrate ausgewählt aus der Gruppe bestehend aus Haar, Haut, harte Oberflächen und Textilien
   oder
- als Mittel zur Verringerung der augenreizenden Wirkung von ein, zwei oder mehr Tensiden ausgewählt aus der Gruppe bestehend aus Sodium Laureth Sulfate, Cocoamidopropyl Betain und Sodium Laurylsulfate.Weitere (Teil-)Aufgaben der vorliegenden Erfindung ergeben sich aus den beigefügten Patentansprüchen und der vorliegenden Beschreibung.

Die vorstehend mit Blick auf die anzugebende Zusammensetzung dargelegten weiteren (Teil-)Aufgaben gelten entsprechend auch für die vorzugsweise anzugebende Verwendung, das anzugebende Verfahren und so weiter.

Nachfolgend werden Stoffe gemäß der IUPAC-Nomenklatur oder gemäß einer anderen, dem Fachmann auf dem Gebiet der Spül- bzw. Reinigungsmittel, beziehungsweise auf dem Gebiet der Tenside geläufigen Nomenklatur bezeichnet. Insbesondere werden nachfolgend solche Stoffe, die auch als Inhaltsstoffe von kosmetischen Mitteln dienen, gegebenenfalls gemäß der INCI-Nomenklatur bezeichnet (INCI = International Nomenclature of Cosmetic Ingredients). INCI-Bezeichnungen finden sich im "International Cosmetic Ingredient Dictionary & Handbook", (15. Aufl., 2014) Herausgeber: Personal Care Products Council.

Nachfolgend werden ausgewählte Tenside auch über ihre entsprechende CAS-Nummer definiert, nämlich: Sodium Laureth Sulfate (CAS-Nr. 68891-38-3), Cocoamidopropyl Betain (CAS-Nr. 147170-44-3), Sodium Laurylsulfate (CAS-Nr. 73296-89-6), Lauryl/Myristyl Glucoside (CAS-Nr. 110615-47-9), PEG-4 Rapeseedamide CAS-Nr. 85536-23-8).

Die vorstehend genannten Aufgaben werden durch Verwendungen gelöst, wie sie in den beigefügten Ansprüchen definiert sind.

Offenbart wird eine Zusammensetzung,
umfassend
ein, zwei oder mehr (d.h. sämtliche) Tenside ausgewählt aus der Gruppe bestehend aus Sodium Laureth Sulfate, Cocoamidopropyl Betain und Sodium Laurylsulfate
sowie
zum Abmildern oder Vermeiden von Augenreizungen N-Methyl-N-((2S,3R,4R,5R)-2,3,4,5,6-pentahydroxyhexyl)-(Z)-octadec-9-enamid sowie ein, zwei oder mehr weitere N-Methyl-N-((2S,3R,4R,5R)-2,3,4,5,6-pentahydroxyhexyl)-Fettsäureamide,
wobei
- das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide größer als 0,75 ist
   und
- das Verhältnis der Gesamtmasse sämtlicher N-Methyl-N-((2S,3R,4R,5R)-2,3,4,5,6-pentahydroxyhexyl)-Fettsäureamide zur Gesamtmasse sämtlicher weiterer Tenside, bei denen es sich nicht um N-Methyl-N-((2S,3R,4R,5R)-2,3,4,5,6-pentahydroxyhexyl)-Fettsäureamide handelt, kleiner ist als 1,
gelöst.

Eine solche vorstehend offenbarte Zusammensetzung umfasst ein, zwei oder mehr (d.h. sämtliche) Tenside ausgewählt aus der Gruppe bestehend aus Sodium Laureth Sulfate, Cocoamidopropyl Betain und Sodium Laurylsulfate, d.h. sie umfasst:
- Sodium Laureth Sulfate, aber weder Cocoamidopropyl Betain noch Sodium Laurylsulfate,
- Cocoamidopropyl Betain, aber weder Sodium Laureth Sulfate noch Sodium Laurylsulfate,
- Sodium Laurylsulfate, aber weder Sodium Laureth Sulfate noch Cocoamidopropyl Betain,
- Sodium Laureth Sulfate und Cocoamidopropyl Betain, aber nicht Sodium Laurylsulfate,
- Sodium Laureth Sulfate und Sodium Laurylsulfate, aber nicht Cocoamidopropyl Betain
- Cocoamidopropyl Betain und Sodium Laurylsulfate, aber nicht Sodium Laureth Sulfate
   oder
- Sodium Laureth Sulfate, Cocoamidopropyl Betain und Sodium Laurylsulfate.

Die Verbindung N-Methyl-N-((2S,3R,4R,5R)-2,3,4,5,6-pentahydroxyhexyl)-(Z)-octadec-9-enamid besitzt die Struktur gemäß der nachfolgenden Formel 1:

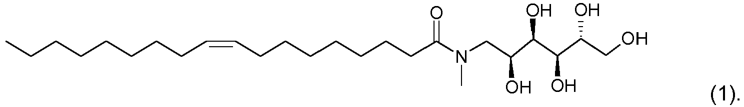

N-Methyl-N-((2S,3R,4R,5R)-2,3,4,5,6-pentahydroxyhexyl)-(Z)-octadec-9-enamid ist das N-Methyl-N-((2S,3R,4R,5R)-2,3,4,5,6-pentahydroxyhexyl)-Fettsäureamid, der (Z)-octadec-9-ensäure, die auch als Ölsäure bezeichnet wird.

Die vorstehend offenbarte Zusammensetzung umfasst zum Abmildern oder Vermeiden von Augenreizungen N-Methyl-N-((2S,3R,4R,5R)-2,3,4,5,6-pentahydroxyhexyl)-(Z)-octadec-9-enamid sowie ein, zwei oder mehr weitere N-Methyl-N-((2S,3R,4R,5R)-2,3,4,5,6-pentahydroxyhexyl)-Fettsäureamide, wobei das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide größer als 0,75 ist. Der Verhältnis der Gesamtmasse der restlichen N-Methyl-N-((2S,3R,4R,5R)-2,3,4,5,6-pentahydroxyhexyl)-Fettsäureamide zu der besagten Gesamtmasse sämtlicher N-Methyl-N-((2S,3R,4R,5R)-2,3,4,5,6-pentahydroxyhexyl)-Fettsäureamide ist folglich kleiner als 0,25.

Unter den in der vorstehend offenbarten Zusammensetzung vorhandenen N-Methyl-N-((2S,3R,4R,5R)-2,3,4,5,6-pentahydroxyhexyl)-Fettsäureamiden überwiegt somit sehr deutlich der Anteil an N-Methyl-N-((2S,3R,4R,5R)-2,3,4,5,6-pentahydroxyhexyl)-(Z)-octadec-9-enamid. Hierdurch wird überraschenderweise die besondere Augenmilde der vorstehend offenbarten Zusammensetzung erreicht. N-Methyl-N-((2S,3R,4R,5R)-2,3,4,5,6-pentahydroxyhexyl)-(Z)-octadec-9-enamid ist bei Einsatz in üblichen Konzentrationsbereichen selbst augenmild, und es ist überraschenderweise auch dazu befähigt, die augenreizende bzw. augenschädigende Wirkung insbesondere der Tenside Sodium Laureth Sulfate, Cocoamidopropyl Betain und Sodium Laurylsulfate abzumildern, und zwar im Sinne einer echten (synergistischen) Wechselwirkung mit diesen.

Dabei ist in der vorstehend offenbarten Zusammensetzung das Verhältnis der Gesamtmasse sämtlicher N-Methyl-N-((2S,3R,4R,5R)-2,3,4,5,6-pentahydroxyhexyl)-Fettsäureamide zur Gesamtmasse sämtlicher weiterer Tenside, bei denen es sich nicht um N-Methyl-N-((2S,3R,4R,5R)-2,3,4,5,6-pentahydroxyhexyl)-Fettsäureamide handelt, kleiner als 1. Mit anderen Worten überwiegt die Gesamtmasse der weiteren Tenside im Vergleich mit der Gesamtmasse der N-Methyl-N-((2S,3R,4R,5R)-2,3,4,5,6-pentahydroxyhexyl)-Fettsäureamide.

Die besondere Augenmilde der vorstehend offenbarten tensidhaltigen Zusammensetzung ist überraschend; sie geht maßgeblich auf die Anwesenheit einer Menge von N-Methyl-N-((2S,3R,4R,5R)-2,3,4,5,6-pentahydroxyhexyl)-(Z)-octadec-9-enamid zurück.

Weder die Augenmilde des in der vorstehend offenbarten Zusammensetzung enthaltenen Tensids N-Methyl-N-((2S,3R,4R,5R)-2,3,4,5,6-pentahydroxyhexyl)-(Z)-octadec-9-enamid selbst noch dessen Fähigkeit, die augenreizende Wirkung anderer Tenside abzumildern, bzw. zu reduzieren, war für den Fachmann vorhersehbar. Insbesondere war für den Fachmann nicht vorhersehbar, dass sich diese technische Wirkung in Zusammensetzungen (wie oben offenbart) zeigt, in denen
- das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide größer als 0,75 ist
   und
- das Verhältnis der Gesamtmasse sämtlicher N-Methyl-N-((2S,3R,4R,5R)-2,3,4,5,6-pentahydroxyhexyl)-Fettsäureamide zur Gesamtmasse sämtlicher weiterer Tenside, bei denen es sich nicht um N-Methyl-N-((2S,3R,4R,5R)-2,3,4,5,6-pentahydroxyhexyl)-Fettsäureamide handelt, kleiner ist als 1.

Darüber hinaus konnte der Fachmann nicht vorhersehen, dass sich die technische Wirkung in Zusammensetzungen (wie oben offenbart) zeigt, die ionische Tenside, insbesondere anionische und amphotere Tenside enthalten.

Die vorstehend offenbarte Zusammensetzung besitzt darüber hinaus weitere hervorragende Eigenschaften. Die Reinigungskraft der vorstehend offenbarten Zusammensetzungen (insbesondere, wenn es sich um ein Handgeschirrspülmittel handelt) ist beispielsweise zumindest ebenso hoch wie die Reinigungskraft einer Vergleichszusammensetzung, die bei ansonsten gleichen Anteilen der gleichen Bestandteile kein N-Methyl-N-((2S,3R,4R,5R)-2,3,4,5,6-pentahydroxyhexyl)-(Z)-octadec-9-enamid enthält. Das erfindungsgemäß verwendete Tensid N-Methyl-N-((2S,3R,4R,5R)-2,3,4,5,6-pentahydroxyhexyl)-(Z)-octadec-9-enamid fungiert selbst als Tensid und besitzt selbst eine hervorragende Reinigungskraft.

N-Methyl-N-((2S,3R,4R,5R)-2,3,4,5,6-pentahydroxyhexyl)-(Z)-octadec-9-enamid ist in der Natur und in Kläranlagen leicht abbaubar, was vermutlich unter anderem auf den Umstand zurückzuführen ist, dass der Fettsäurerest der Verbindung linear (und nicht etwa verzweigt) ist.

Eine Zusammensetzung (wie oben offenbart) ist vorzugsweise herstellbar durch ein Verfahren mit folgenden Schritten:
a) Bereitstellen oder Herstellen eines Startmaterials ausgewählt aus der Gruppe bestehend aus
   High Oleic Sonnenblumenöl,
   Mischung von Fettsäuren aus High Oleic Sonnenblumenöl und/oder deren Salzen, Mischung von Estern von Fettsäuren aus High Oleic Sonnenblumenöl,
   Mischung von Fettsäurechloriden von Fettsäuren aus High Oleic Sonnenblumenöl und deren Mischungen,
b) Umsetzen des Startmaterials mit (2R,3R,4R,5S)-6-(Methylamino)hexan-1,2,3,4,5-pentol.

Die vorstehend offenbarte Zusammensetzung basiert somit vorzugsweise auf High Oleic Sonnenblumenöl, einem Öl, welches aus der High Oleic Sonnenblume gewonnen werden kann. Der Begriff "High Oleic Sonnenblumenöl" ist dem Fachmann bekannt; er bezeichnet insbesondere Sonnenblumenöle mit einem Anteil an Ölsäure im Bereich von 70 - 92 Gew.-%, bezogen auf den Anteil der Gesamtmenge an ungebundenen oder in Form von Triglyceriden gebundenen Fettsäuren im Sonnenblumenöl. Die zusätzliche Verwendung anderer Pflanzenöle ist im Rahmen der vorliegenden Erfindung, d.h. zur Lösung der zugrundeliegenden technischen Aufgabe, nicht erforderlich und regelmäßig schon aus Gründen der Nachhaltigkeit nicht erwünscht. Es ist bevorzugt, wenn in einer Zusammensetzung (wie oben offenbart) das Fettsäuremuster der N-Methyl-N-((2S,3R,4R,5R)-2,3,4,5,6-pentahydroxyhexyl)-Fettsäureamide mit dem Fettsäuremuster eines High Oleic Sonnenblumenöls übereinstimmt. Der Begriff Fettsäuremuster (auch Fettsäurezusammensetzung, Fettsäurespektrum oder Fettsäureprofil genannt) bezeichnet das Mengenverhältnis der unterschiedlichen Fettsäuren in einem Fett oder Öl; der Begriff wird im Rahmen des vorliegenden Textes auch für Fettsäureamidmischungen, Fettsäureestermischungen und Fettsäurechloridmischungen verwandt.

Zu bevorzugten Herstellverfahren siehe auch unten; die dort gemachten Angaben gelten entsprechend für bevorzugte vorstehend offenbarte Zusammensetzungen.

### (2R,3R,4R,5S)-6-(Methylamino)hexan-1,2,3,4,5-pentol ist eine Verbindung der Formel 2

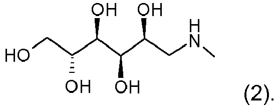

Bevorzugt ist eine vorstehend offenbarte Zusammensetzung weder eine O/W-Emulsion noch eine W/O-Emulsion; ganz besonders bevorzugt ist eine vorstehend offenbarte Zusammensetzung gar keine Emulsion. Bevorzugte vorstehend offenbarte Zusammensetzungen sind einphasig oder stellen die kontinuierliche Phase einer Suspension dar, welche als suspendierten Feststoff vorzugsweise Abrasivmittel wie Marmormehl umfasst.

Die Verbindung (2R,3R,4R,5S)-6-(Methylamino)hexan-1,2,3,4,5-pentol der Formel 2 wird auch als N-Methyl-D-glucamin bezeichnet und ist von der Glucose abgeleitet. Glucose ist ein aus einer nachhaltigen Rohstoffquelle stammender Wertstoff, der in der Regel durch enzymatische Verzuckerung von stärkehaltigen Agrarprodukten wie z.B. Mais gewonnen wird. Durch Umsetzung von N-Methyl-D-glucamin mit (Z)-octadec-9-ensäure (Ölsäure), (Z)-octadec-9-ensäurechlorid oder (Z)-octadec-9-ensäureestern, insbesondere (Z)-octadec-9-ensäuremethylester, wird N-Methyl-N-((2S,3R,4R,5R)-2,3,4,5,6-pentahydroxyhexyl)-(Z)-octadec-9-enamid (Formel 1) erhalten. Die Verbindung wird in manchen Fällen auch als (Z)-9-Octadecensäure-N-methyl-N-glucamid oder als 1-Deoxy-1-(methyl-((Z)-9-octadecenoyl)amino)-D-Glucitol bezeichnet. Spezifische Fettsäure-N-alkylpolyhydroxyalkylamide sind beispielsweise aus DE 195 29 907 A1 und WO2013/178669 A2 bekannt. Zum Gebiet der Zuckertenside vergleiche auch die Dissertation "Tenside, Polymerbausteine und Polymere aus Nachwachsenden Rohstoffen durch Epoxidringöffnungen mit Aminen und enzymatische Polykondensationen" von F. Brüse, RWTH Aachen, 2003.

Die Konfiguration des N-Methyl-D-glucamin (Formel 2) verändert sich bei der Synthese nicht, gemäß den IUPAC-Richtlinien kehrt sich aber die Nummerierung der Kohlenstoffatome um. Im (2R,3R,4R,5S)-6-(Methylamino)hexan-1,2,3,4,5-pentol der Formel 2 hat der an Stickstoff gebundene Kohlenstoff die Nummer 6, wohingegen im N-Methyl-N-((2S,3R,4R,5R)-2,3,4,5,6-pentahydroxyhexyl)-(Z)-octadec-9-enamid der entsprechende über die Amid-Funktion an die Hauptkette gebundene Kohlenstoff die Nummer 1 besitzt.

Bevorzugt ist eine vorstehend offenbarte Zusammensetzung,
wobei der Gesamtanteil an N-Methyl-N-((2S,3R,4R,5R)-2,3,4,5,6-pentahydroxyhexyl)-Fettsäureamiden in der Zusammensetzung kleiner ist als 5 Gew.-%.

In diesen bevorzugten Zusammensetzungen reduziert N-Methyl-N-((2S,3R,4R,5R)-2,3,4,5,6-pentahydroxyhexyl)-(Z)-octadec-9-enamid die augenreizende Wirkung von Tensiden ausgewählt aus der Gruppe bestehend aus Sodium Laureth Sulfate, Cocamidopropyl Betain, und Sodium Laurylsulfate besonders effektiv, ohne dass es dabei als ein mengenmäßiger Hauptbestandteil der Zusammensetzung eingesetzt wird.

Darüber hinaus bevorzugt ist eine vorstehend offenbarte Zusammensetzung (vorzugsweise eine Zusammensetzung wie vorstehend als bevorzugt bezeichnet), wobei das Verhältnis der Gesamtmasse sämtlicher N-Methyl-N-((2S,3R,4R,5R)-2,3,4,5,6-pentahydroxyhexyl)-Fettsäureamide zur Gesamtmasse sämtlicher weiterer Tenside, bei denen es sich nicht um N-Methyl-N-((2S,3R,4R,5R)-2,3,4,5,6-pentahydroxyhexyl)-Fettsäureamide handelt, kleiner ist als 0,5, bevorzugt kleiner als 0,2.

Die physikalisch-chemischen Eigenschaften solcher bevorzugten Zusammensetzungen sowie insbesondere ihre spezifische Wirksamkeit gegen bestimmte Verunreinigungen werden nur im geringen Maße von den Eigenschaften der N-Methyl-N-((2S,3R,4R,5R)-2,3,4,5,6-pentahydroxyhexyl)-Fettsäureamide beeinflusst; diese Eigenschaften werden vielmehr maßgeblich durch die Wahl der in überwiegender Menge eingesetzten weiteren Tenside bestimmt, bei denen es sich nicht um N-Methyl-N-((2S,3R,4R,5R)-2,3,4,5,6-pentahydroxyhexyl)-Fettsäureamide handelt. Zu diesen weiteren Tensiden zählen dabei insbesondere auch die in der vorstehend offenbarten Zusammensetzung vorhandenen ein, zwei oder mehr Tenside ausgewählt aus der Gruppe bestehend aus Sodium Laureth Sulfate, Cocoamidopropyl Betain und Sodium Laurylsulfate.

Bevorzugt ist eine vorstehend offenbarte Zusammensetzung (vorzugsweise wie vorstehend als bevorzugt bezeichnet), zusätzlich umfassend ein oder mehr Tenside ausgewählt aus der Gruppe bestehend aus Lauryl/Myristyl Glucoside und PEG-4 Rapeseedamide.

Derartig bevorzugte vorstehend offenbarte Zusammensetzungen sind besonders augenmild und besitzen dabei dennoch eine hervorragende Reinigungskraft, insbesondere, wenn die Zusammensetzung als Spül- oder Reinigungsmittel ausgestaltet ist. Lauryl/Myristyl Glucoside und PEG-4 Rapeseedamide sind nichtionische Tenside, deren Vorliegen die Aktivität von N-Methyl-N-((2S,3R,4R,5R)-2,3,4,5,6-pentahydroxyhexyl)-(Z)-octadec-9-enamid zum Abmildern oder Vermeiden von Augenreizungen nicht negativ beeinflusst und deren eigene augenreizende Wirkung durch N-Methyl-N-((2S,3R,4R,5R)-2,3,4,5,6-pentahydroxyhexyl)-(Z)-octadec-9-enamid gemildert wird. Lauryl/Myristyl Glucoside und PEG-4 Rapeseedamide können unter Verwendung nachhaltiger, erneuerbarer Rohstoffquellen (auf Pflanzenbasis) synthetisiert werden, sodass sich in einer bevorzugten vorstehend offenbarten Zusammensetzung ein hoher Anteil an Tensiden einstellen lässt, die nicht erdölbasiert sind.

Besonders bevorzugt ist auch eine vorstehend offenbarte Zusammensetzung (vorzugsweise wie vorstehend als bevorzugt bezeichnet), umfassend zumindest zwei, vorzugsweise aber zumindest drei oder vier Tenside ausgewählt aus der Gruppe bestehend aus Sodium Laureth Sulfate, Cocoamidopropyl Betain, Lauryl/Myristyl Glucoside, Sodium Laurylsulfate und PEG-4 Rapeseedamide. Dabei ist zumindest ein Tensid ausgewählt aus der Gruppe bestehend aus Sodium Laureth Sulfate, Cocoamidopropyl Betain und Sodium Laurylsulfate.

Derartige bevorzugte vorstehend offenbarte Zusammensetzungen sind besonders augenmild und besitzen dabei dennoch eine hervorragende Reinigungskraft. Bedingt durch die unterschiedlichen physikalisch-chemischen Eigenschaften und insbesondere das unterschiedliche Polaritätsprofil der genannten Tenside lassen sich bevorzugte vorstehend offenbarte Zusammensetzungen herstellen, die effektiv an die vorgesehene Anwendung angepasst sind, während gleichzeitig die überraschende Wirkung des N-Methyl-N-((2S,3R,4R,5R)-2,3,4,5,6-pentahydroxyhexyl)-(Z)-octadec-9-enamid besonders effizient ausgenutzt wird.

Bevorzugt ist eine PEG-4 Rapeseedamide umfassende vorstehend offenbarte Zusammensetzung (vorzugsweise wie vorstehend als bevorzugt bezeichnet).

Die augenreizende Wirkung solcher bevorzugter Zusammensetzungen ist effektiv verringert, ihre Reinigungskraft ist jedoch besonders hoch, so dass sich ein besonders günstiges Verhältnis zwischen der Reinigungskraft und der Augenmilde ergibt. Solche vorstehend offenbarten Zusammensetzungen sind unter dem Gesichtspunkt der Nachhaltigkeit insbesondere auch deshalb bevorzugt, weil Rapsöl die Basis für PEG-4 Rapeseedamide ist, also weder Palm- bzw. Palmkernöl noch Kokosöl.

Eine bevorzugte vorstehend offenbarte Zusammensetzung (vorzugsweise wie vorstehend als bevorzugt bezeichnet) umfasst zusätzlich ein, zwei oder mehr weitere Tenside, wobei diese weiteren Tenside unabhängig voneinander ausgewählt sind aus der Gruppe bestehend aus anionischen Tensiden, kationischen Tensiden, amphoteren Tensiden und nichtionischen Tensiden, wobei die anionischen Tenside vorzugsweise ausgewählt sind aus der Gruppe bestehend aus Alkylcarboxylaten, Alkylethercarboxylaten, Alkenylcarboxylaten, Alkylphosphaten, Alkenylphosphaten, Alkylphosphonaten, Alkenylphosphonaten, Sulfosuccinaten, Alkylsulfoacetaten, Alkylisethionaten, Tauriden, Alkylarylsulfonaten, Alkylsulfonaten, Alkenylsulfonaten, Alkylsulfaten, Alkenylsulfaten, Alkylethersulfaten und Alkenylethersulfaten,
und/oder
wobei die kationischen Tenside vorzugsweise ausgewählt sind aus der Gruppe bestehend aus primären, sekundären, tertiären oder quartären Alkylammoniumsalzen mit Kationen der Formel [(R1)(R2)(R3)(R4)N]+, wobei R1 bis R4 unabhängig voneinander (i) verzweigte oder unverzweigte, (ii) gesättigte oder ungesättigte, (iii) unsubstituierte, einfach oder mehrfach substituiert Alkyl- bzw. Alkenylkette sind,
und/oder
wobei die amphoteren Tenside vorzugsweise ausgewählt sind aus der Gruppe bestehend aus Imidazolcarboxylaten, Amphoacetaten, Amidoaminen, Amidobetainen, Sulfobetainen, Sultainen und Phosphobetainen,
und/oder
wobei die nichtionischen Tenside vorzugsweise ausgewählt sind aus der Gruppe bestehend aus Fettsäure-N-alkylpolyhydroxyalkylamide, Fettalkoholethoxylaten, Fettalkoholpropoxylaten, Fettalkoholbutoxylaten, Alkylarylethoxylaten, Aminoxiden, Alkylpolyglycosiden, Alkylglycosiden, N-Alkylpyrrolidonen und Saponinen.

In solchen bevorzugten vorstehend offenbarten Zusammensetzungen bewirkt die Verbindung N-Methyl-N-((2S,3R,4R,5R)-2,3,4,5,6-pentahydroxyhexyl)-(Z)-octadec-9-enamid eine Verringerung der augenreizenden Effekte der zusätzlich eingesetzten ein, zwei oder mehr weiteren Tenside. Die Reinigungskraft einer solchen bevorzugten vorstehend offenbarten Zusammensetzung ist dabei im Vergleich mit einer Zusammensetzung, die bei ansonsten gleichen Anteilen der gleichen Bestandteile kein N-Methyl-N-((2S,3R,4R,5R)-2,3,4,5,6-pentahydroxyhexyl)-(Z)-octadec-9-enamid enthält, nicht oder zumindest nicht nennenswert reduziert, da N-Methyl-N-((2S,3R,4R,5R)-2,3,4,5,6-pentahydroxyhexyl)-(Z)-octadec-9-enamid selbst als Tensid fungiert und selbst eine hervorragende Reinigungskraft besitzt.

Bedingt durch die unterschiedlichen physikalisch-chemischen Eigenschaften und insbesondere das unterschiedliche Polaritätsprofil der genannten Tenside lassen sich bevorzugte vorstehend offenbarte Zusammensetzungen herstellen, die effektiv an die vorgesehene Anwendung angepasst sind, während gleichzeitig die überraschende Wirkung des N-Methyl-N-((2S,3R,4R,5R)-2,3,4,5,6-pentahydroxyhexyl)-(Z)-octadec-9-enamid besonders effizient ausgenutzt wird.

Bevorzugt ist eine vorstehend offenbarte Zusammensetzung (vorzugsweise wie vorstehend als bevorzugt bezeichnet), zur Verwendung als Handgeschirrspülmittel, vorzugsweise als augenmildes Handgeschirrspülmittel, umfassend
einen Gesamtanteil sämtlicher N-Methyl-N-((2S,3R,4R,5R)-2,3,4,5,6-pentahydroxyhexyl)-Fettsäureamide im Bereich von 0,5 bis 1,5 Gew.-% und
einen Anteil an PEG-4 Rapeseedamide im Bereich von 0 bis 1 Gew.-%, bevorzugt > 0 bis 1 Gew.-%, besonders bevorzugt 0,05 bis 0,5 Gew.-%.

Der angegebene Gesamtanteil sämtlicher N-Methyl-N-((2S,3R,4R,5R)-2,3,4,5,6-pentahydroxyhexyl)-Fettsäureamide ist unabhängig von der An- oder Abwesenheit des PEG-4 Rapeseedamide bevorzugt; die Anwesenheit von PEG-4 Rapeseedamide ist jedoch vorteilhaft, wobei Konzentrationen in den vorstehend angegebenen Bereichen wiederum bevorzugt sind. Insbesondere in den angegebenen Konzentrationsbereichen ergibt sich eine besonders augenmilde Zusammensetzung mit guter Reinigungsleistung.

Die vorstehend beschriebenen Vorteile sind für die angegebenen Konzentrationsbereiche besonders ausgeprägt, so dass diese bevorzugten Zusammensetzungen für die Verwendung als Handgeschirrspülmittel, insbesondere als augenmildes Handgeschirrspülmittel in besonderer Weise geeignet sind. Bevorzugt ist eine vorstehend offenbarte Zusammensetzung (vorzugsweise wie vorstehend als bevorzugt bezeichnet), zur Verwendung als Handgeschirrspülmittel, vorzugsweise als augenmildes Handgeschirrspülmittel, umfassend
3 bis 25 Gew.-%, bevorzugt 4 bis 15 Gew.-% Sodium Laureth Sulfate und/oder
0,5 bis 8 Gew.-%, bevorzugt 0,9 bis 5 Gew.-% Cocoamidopropyl Betain und/oder
0,5 bis 8 Gew.-%, bevorzugt 0,9 bis 5 Gew.-% Lauryl/Myristyl Glucoside und/oder
0,5 bis 8 Gew.-%, bevorzugt 0,9 bis 5 Gew.-% Sodium Laurylsulfate.

Vorzugsweise sind hierbei sämtliche der vier genannten Tenside in den jeweils angegebenen Konzentrationsbereichen anwesend. Aber auch bei Anwesenheit von lediglich einem, zwei oder drei der genannten Tenside sind die die angegebenen Konzentrationsbereiche bevorzugt. Bei gleichzeitiger Anwesenheit von PEG-4 Rapeseedamide sind die vorstehend für dieses Tensid genannten generell bevorzugten Konzentrationsbereiche wiederum bevorzugt.

Ganz besonders bevorzugt ist somit eine vorstehend offenbarte Zusammensetzung (vorzugsweise wie vorstehend als bevorzugt bezeichnet), zur Verwendung als Handgeschirrspülmittel, vorzugsweise als augenmildes Handgeschirrspülmittel, umfassend
einen Gesamtanteil sämtlicher N-Methyl-N-((2S,3R,4R,5R)-2,3,4,5,6-pentahydroxyhexyl)-Fettsäureamide im Bereich von 0,5 bis 1,5 Gew.-%,
einen Anteil an PEG-4 Rapeseedamide im Bereich von 0 bis 1 Gew.-%, bevorzugt > 0 bis 1 Gew.-%, besonders bevorzugt 0,05 bis 0,5 Gew.-%,
einen Anteil an Sodium Laureth Sulfate im Bereich von 3 bis 25 Gew.-%, bevorzugt 4 bis 15 Gew.-%,
einen Anteil an Cocoamidopropyl Betain im Bereich von 0,5 bis 8 Gew.-%, bevorzugt 0,9 bis 5 Gew.-%,
einen Anteil an Lauryl/Myristyl Glucoside im Bereich von 0,5 bis 8 Gew.-%, bevorzugt 0,9 bis 5 Gew.-% und
einen Anteil an Sodium Laurylsulfate im Bereich von 0,5 bis 8 Gew.-%, bevorzugt 0,9 bis 5 Gew.-%.

Bei Einhaltung der jeweils angegebenen Konzentrationsbereiche ergibt sich eine Zusammensetzung mit hoher Reinigungskraft und dennoch besonderer Augenmilde, für die sich die Wirkung des N-Methyl-N-((2S,3R,4R,5R)-2,3,4,5,6-pentahydroxyhexyl)-(Z)-octadec-9-enamid besonders deutlich zeigt. Vorzugsweise umfasst eine solche Zusammensetzung keine weiteren Tenside oder weitere Tenside in einer Gesamtmenge von nicht mehr als 5 Gew.-%, vorzugsweise nicht mehr als 3 Gew.-%.

Bevorzugt ist eine vorstehend offenbarte Zusammensetzung (vorzugsweise wie vorstehend als bevorzugt bezeichnet), zusätzlich umfassend ein, zwei oder mehr weitere Bestandteile, wobei die Bestandteile unabhängig voneinander ausgewählt sind aus der Gruppe bestehend aus Lösungsmitteln, Hydrotropen, pH-Regulatoren, Komplexiermitteln, Salzen, rheologischen Additiven, Enzymen, Vitaminen, Konservierungsmittel, UV-Filtern, Antioxidantien, Trübungsmitteln, Farbstoffen und Riechstoffen.

Hierbei ist vorzugsweise zumindest eines der Lösungsmittel ausgewählt aus der Gruppe bestehend aus Aqua (Wasser), Alcohol (Ethanol), Butoxyethanol, Butoxyisopropanol, Butoxypropanol, n-Butyl Alcohol, t-Butyl Alcohol, Butyloctanol, Dimethyl Ether, Ethoxyethanol, Ethyl Hexanediol, Hexanediol, 1,2,6-Hexanetriol, Hexyl Alcohol, Isobutoxypropanol, Isopentyldiol, Isopropyl, Alcohol (iso-Propanol), 3-Methoxybutanol, Methoxyethanol, Methoxyisopropanol, Methoxymethylbutanol, Methyl Alcohol, Methyl Hexyl Ether, Methylpropanediol, Propanediol, Propyl Alcohol (n-Propanol), Tetrahydrofurfuryl Alcohol, Benzylalcohol, Butylacetat, Methyldiglycol, Ethyldiglycol, Propyldiglycol, Butyldiglycol, Propylenglycolmethylether, Propylenglycolethylether, Propylenglycolpropylylether, Propylenglycolbutylether, Dipropylenglycolmethylether, Dipropylenglycolethylether, Dipropylenglycolpropylether; Dipropylenglycolbutylether, Phenoxyethanol, Phenoxypropanol, 2,2-Dimethyl-1,3-dioxolan-4-methanol und Trimethylhexanol.

Hierbei ist vorzugsweise zumindest eines der Hydrotrope ausgewählt aus der Gruppe bestehend aus Natrium-3,5-xylolsulfonat, Kalium-3,5-xylolsulfonat, Ammonium-3,5-xylolsulfonat, Natriumcumolsulfonat, Kaliumcumolsulfonat, Ammoniumcumolsulfonat, Natriumalkylethersulfat, Kaliumalkylethersulfat, Ammoniumalkylethersulfat, Natrium-2-ethylhexylsulfat, Kalium-2-ethylhexylsulfat, Ammonium-2-ethylhexylsulfat, Natriummonoalkylphosphat, Kaliummonoalkylphosphat, Ammoniummonoalkylphosphat, Natrium-2-ethylhexyliminodipropionat, Kalium-2-ethylhexyliminodipropionat, Ammonium-2-ethylhexyliminodipropionat, Dinatriumdodecyldiphenyloxiddisulfonat, Dikaliumdodecyldiphenyloxid-disulfonat, Diammoniumdodecyldiphenyloxiddisulfonat und Harnstoff.

Hierbei ist vorzugsweise zumindest einer der pH-Regulatoren ausgewählt aus der Gruppe bestehend aus Säuren und Basen, vorzugsweise Ameisensäure, Essigsäure, Zitronensäure, Glycolsäure, Milchsäure, Bernsteinsäure, Adipinsäure, Apfelsäure, Weinsäure, Salicylsäure, Gluconsäure, Alkalihydroxid, Erdalkalihydroxid, Alkalicarbonat, Erdalkalicarbonat und Ammoniak.

Hierbei ist vorzugsweise zumindest eines der Komplexiermittel ausgewählt aus der Gruppe bestehend aus Aminotrimethylene, Phosphonsäure, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, EthylenediamineA/,A/'-disuccinic acid (EDDS), Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Phosphonobutane tricarboxylic acid (PBTC), Pentasodium Ethylenediamine Tetramethylene Phosphonate, Pentasodium Pentetate, Pentasodium Triphosphate, Pentetic Acid, (DTPA), PhyticAcid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA Polyphosphate, Tetrahydroxyethyl Ethylenediamine, Tetrahydroxypropyl Ethylenediamine, Tetrapotassium Etidronate, Tetrasodium Iminodisuccinate (IDS), Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA, Glutaminsäure, N,N-Diacetat Tetranatriumsalz, Iminodisuccinat Tetranatriumsalz und Trisodium Phosphate.

Hierbei ist vorzugsweise zumindest eines der Salze ausgewählt aus der Gruppe bestehend aus Natriumchlorid, Kaliumchlorid, Calciumchlorid und Magnesiumchlorid.

Hierbei ist vorzugsweise zumindest eines der rheologischen Additive ausgewählt aus der Gruppe bestehend aus Polyacrylaten, Carboxymethylcellulose, Hydroxyethylcellulose, Hydroxypropylcellulose, Kernmehlether, Kieselsäuren, Schichtsilikaten, Montmorilloniten, Zeolithen, Agar-Agar, Carrageen, Xanthan, Traganth, Gummi arabicum, Alginate, Pektine, Polyosen, Guar-Mehl, Johannisbrotbaumkernmehl, Stärke, Dextrine, Gelatine und Casein.

Hierbei ist vorzugsweise zumindest eines der Enzyme ausgewählt aus der Gruppe bestehend aus Pectinasen, Proteasen, Lipasen, Amylasen, Mannanasen, Cellulasen und Hydrolasen.

Hierbei ist vorzugsweise zumindest eines der Vitamine ausgewählt aus der Gruppe bestehend aus fettlöslichen Vitaminen, wasserlöslichen Vitaminen und Provitaminen, vorzugsweise Vitamin C, Vitamin E, Vitamin B5 und Provitamin B5

Hierbei ist vorzugsweise zumindest eines der Konservierungsmittel ausgewählt aus der Gruppe bestehend aus Phenoxyethanol, Formaldehyd, Paraformaldehyd, Parabene, Pentandiol, Sorbinsäure und deren Salze, Benzoesäure und deren Salze, Propionsäure und deren Salze, Salicylsäure und deren Salze, 2-Hydroxidiphenyl und deren Salze, 4-Hydroxybenzoesäure und deren Salze, Dehydracetsäure und deren Salze, Ameisensäure und deren Salze, Milchsäure und deren Salze Dibromhexamidin und seiner Salze, 10-Undecylensäure und deren Salze, 5-Amino-1,3-bis (2-ethylhexyl)-5-methyl-hexahy-dropyrimidin, Chlorobutanolum, 2,4-Dichlorbenzylalkohol, 4-Chlor-m-cresol, 2,4,4'-Trichlor-2'-hydroxy-diphenylether, Imidazolidinylharnstoff, 2-Phenoxy-ethanol, Poly(hexamethylendiguanid)-hydrochlorid, Hexamethylentetramin, 1-(3-Chlorallyl)-3,5,7-triaza-1-azonia-adamantanchlorid, 1,3-Bis-(hydroxymethyl)-5,5- dimethyl-2,4-imidazolidindion, Benzylalkohol, 1-Hydroxy-4-methyl-6-(2,4,4-trimethylpentyl)-2-pyridon, 2,2'-Methylen-bis (6-brom-4-chlorphenol), 3-Methyl-4-(1- methylethyl)phenol, 2-Benzyl-4-chlorphenol, 2-Chloracetamid, 1-Phenoxy-propan-2-ol, N-Alkyl (C12 - C22) trimethylammoniumbromid, N-Alkyl(C12-C22)trimethylammoniumchlorid, 4,4-Dimethyl-1,3-oxazolidin, N-Hydroxymethyl-N-1,3-di(hydroxymethyl)-2,5- dioxoimidazolidin- 4-yl)-N'-hydroxymethyl-harnstoff, 1,6-Bis(4-amidinophenoxy)-n-hexan, Pentan-1,5-dial, 5-Ethyl-1-aza-3,7-dioxabicyclo[3.3.0]octan, 3-(4-Chlorphenoxy)-1,2-propandiol, Natriumhydroxymethylaminoacetat, Benzethonium-chlorid, Benzalkoniumchlorid, Benzalkoniumbromid, Benzalkoniumsacharinat, Benzylhemiformal, lodopropinylbutylcarbamat, Ethyl-N-dodecanoyl-L-argininhydrochlorid, 2-Brom-2-nitropropan-1,3-diol, 4-Hydroxybenzoesäure, 5-Brom-5-nitro-1,3-dioxan, Ammoniumbenzoat, Ammoniumbisulfit, Ammoniumhydrogensulfit, Ammoniumpropionat, Ammoniumsulfit, Benzalkoniumbromid, Benzalkoniumchlorid, Benzethoniumchlorid, Benzoesäure, Benzylalkohol, Bromchlorophen, Benzoesäure-Butylester, 4-Hydroxybenzoesäurebutylester, Calciumbenzoat, Calcium 4-Hydroxybenzoat, Calciumpropionat, Calciumsalicylat, Calciumsorbat, Cetrimoniumchlorid, Chlorhexidin, Chloracetamid, Chlorbutanol, 4-Chlor-2-(phenylmethyl)phenol, 3-(4-Chlorphenoxy)-1,2-propandiol, 1-(4-Chlorphenoxy)-1-(1H-imidazol-1-yl)-3,3-dimethyl-2-butanon, Dehydracetsäure, 3-Acetyl-6-methyl-2H-pyran-2,4(3H)-dion, Diazolidinylharnstoff, 1,3-Bis(hydroxymethyl)-5,5-dimethylimidazolidin-2,4-dion, Benzoesäure-Ethylester, N-Lauroyl-L-arginin-ethylester-hydroch lorid, 4-Hydroxybenzoesäureethylester, Methanal, Ameisensäure, Milchsäure, Glutaral, Hexamidin, Imidazolidinyl-Harnstoff, 3-lod-2-propinylbutylcarbamat, Benzoesäure-Isobutylester, Benzoesäure-Isopropylester, 4-Isopropyl-m-cresol, Magnesiumbenzoat, Magnesiumpropionat, Magnesiumsalicylat, Monoethanolaminbenzoat, Benzoesäure-Methylester, 5-Chlor-2-methyl-2H-isothiazol-3-on, 2-Methyl-2H-isothiazol-3-on, 4-Hydroxybenzoesäuremethylester, o-Phenylphenol, 4-Chlor-3-methylphenol, 2-Phenoxyethanol, Phenoxyisopropanol, Benzoesäure-Phenylester, Pirocton-Olamin, Polyhexamethylenbiguanid (PHMB), Kaliumbenzoat, 4-Hydroxybenzoesäurebutylester (Kaliumsalz), 4-Hydroxybenzoesäureethylester (Kaliumsalz), Dikaliumdisulfit, 4-Kaliumsalz des Hydroxybenzoesäuremethylester, Kalium 4-Hydroxybenzoat,Kaliumpropionat, Kaliumsalz des 4-Hydroxybenzoesäurepropylester, Kaliumsalicylat, Kaliumsorbat, Kaliumsulfit, Propionsäure, Benzoesäure-Propylester, 4-Hydroxybenzoesäurepropylester, Natriumbenzoat, Natriumhydrogensulfit, Natriumsalz des 4-Hydroxybenzoesäurebutylester, Natriumsalz des 4-Hydroxybenzoesäureethylester, Natriumformiat, Natriummetabisulfit, Natriumsalz des 4-Hydroxybenzoesäuremethylester, Natrium 4-Hydroxybenzoat, Natriumpropionat, Natriumsalz des 4-Hydroxybenzoesäurepropylester, Natriumsalicylat, Natriumsorbat, Natriumsulfit, Sorbinsäure, 2,4-Hexadiensäure, Stearalkoniumchlorid, 1-(4-Chlorphenyl)-3-(3,4-dichlorphenyl)harnstoff, 5-Chlor-2-(2,4-dichlorphenoxy)phenol, Natriumpyrithion und Zinkpyrithion.

Hierbei ist vorzugsweise zumindest einer der UV-Filter ausgewählt aus der Gruppe bestehend aus UVA-Filtern und UVB-Filtern,
vorzugsweise Derivaten des 3-Benzylidencampher, Derivaten der 4-Aminobenzoesäure, Estern der Zimtsäure, Estern der Salicylsäure, Derivaten des Benzophenons, Estern der Benzalmalonsäure, Triazinderivate, 2-Phenylbenzimidazol-5-sulfonsäure, Sulfonsäure-Derivate von Benzophenonen, Sulfonsäure-Derivate des 3-Benzylidencamphers, Propan-1,3-dione, Derivate des Ketotricyclo(5.2.1.0)decans, Derivate des Dibenzoylmethans und unlösliche Lichtschutzpigmente,
besonders vorzugsweise 3-(4-Methylbenzyliden)- campher, 4-(Dimethylamino)-benzoesäure(2-ethylhexyl)ester, 4-(Dimethylamino)benzoësäureamylester, 4-Methoxyzimtsäure(2-ethylhexyl)ester, 4-Methoxyzimtsäureisopentylester, Salicylsäure(2-ethylhexyl)ester, Salicylsäure(4-isopropylbenzyl)ester, Salicylsäurehomomenthylester, 2-Hydroxy-4-methoxybenzophenon, 2-Hydroxy-4-methoxy-4'-methylbenzophenon, 2,2'-Dihydroxy-4-methoxybenzophenon, 4-Methoxybenzalmalonsäuredi(2-ethylhexyl)ester, 2,4,6-Trianilino-(p-carbo-2'-ethyl-1'-hexyloxy)-1,3,5-triazin, Natrium 2-Phenylbenzimidazol-5-sulfonat, Kalium 2-Phenylbenzimidazol-5-sulfonat, Triethanolammonium 2-Phenylbenzimidazol-5-sulfonat, 2-Hydroxy-4-methoxybenzophenon-5-sulfonsäure, Natrium 2-Hydroxy-4-methoxybenzophenon-5-sulfonat, Kalium 2-Hydroxy-4-methoxybenzophenon-5-sulfonat, Triethanolammonium 2-Hydroxy-4-methoxybenzophenon-5-sulfonat, 4-(2-Oxo-3-bornylidenmethyl)benzolsulfonsäure, Natrium 4-(2-Oxo-3-bornylidenmethyl)benzolsulfonat, Kalium 4-(2-Oxo-3-bornylidenmethyl)benzolsulfonat, Triethanolammonium 4-(2-Oxo-3-bornylidenmethyl)benzolsulfonat, 2-Methyl-5-(2-oxo-3-bornylidenmethyl)sulfonsäure, Natrium 2-Methyl-5-(2-oxo-3-bornylidenmethyl)sulfonat, Kalium 2-Methyl-5-(2-oxo-3-bornylidenmethyl)sulfonat, Triethanolammonium 2-Methyl-5-(2-oxo-3-bornylidenmethyl)sulfonat und 1-(4-tert.-Butylphenyl)-3-(4'methoxyphenyl)propan-1,3-dion, 4-tert.-Butyl-4'-methoxydibenzoyl-methan, 1-Phenyl-3-(4'-isopropylphenyl)-propan-1,3-dion, Zinkoxid, Eisenoxid, Titanoxid, Zirkonoxid, Manganoxid, Aluminiumoxid und Ceroxid.

Hierbei ist vorzugsweise zumindest eines der Antioxidantien ausgewählt aus der Gruppe bestehend aus substituierten Phenolen, Hydrochinon, Brenzcatechine, aromatischen Aminen, organischen Sulfiden, Polysulfiden, Dithiocarbamaten, Vitamin C und seinen Derivaten, Vitamin E und seinen Derivaten, Phosphiten und Phosphonaten, insbesondere Hydrochinonen, 2,6 di-tert.-butyl-p-cresol, 2-tert.-butyl-4-hydroxyanisol, 3-tert.-butyl-4-hydroxyanisol, 4,4'thiobis(6-tert.-butyl-m-cresol) und 2-methyl-4,6-dinonyl phenol.

Hierbei umfasst der Ausdruck Trübungsmittel gemäß fachmännischem Verständnis auch solche Verbindungen, die gelegentlich als Perlglanzmittel bezeichnet werden und umfasst allgemein Emulsionen und Dispersionen synthetischer und nativer makromolekularer Substanzen. Hierbei ist vorzugsweise zumindest eines der Trübungsmittel ausgewählt aus der Gruppe bestehend aus Calciumsulfat, Schichtsilikaten, 2-Hydroxyethylpalmitat, 2(2-Hydroxyethoxy)ethylstearat, Ethylenglycolstearat, Ethylenglycol-Distearat Propylenglycol-Distearat, Propylenglycol-Stearat, Polystyrol, Derivaten des Polystyrols, Polyacrylat und Derivaten des Polyacrylats.

Hierbei ist vorzugsweise zumindest einer der Farbstoffe ausgewählt aus der Gruppe bestehend aus wasserlöslichen natürlichen und synthetischen Farbstoffen und Pigmenten, die für Lebensmittel und/oder Kosmetika zugelassen sind gemäß Lebens- und Futtermittelgesetzbuch (LFGB, E-Nummern Liste) und/oder gemäß Anhang IV der Verordnung (EG) Nr. 1223/2009 des europäischen Parlaments und des Rates vom 30. November 2009 über kosmetische Mittel.

Hierbei ist vorzugsweise zumindest einer der Riechstoffe ausgewählt aus der Gruppe bestehend aus natürlichen und synthetischen Riechstoffen,
vorzugsweise Extrakte von Blüten (Lilie, Lavendel, Rosen, Jasmin, Neroli, Ylang-Ylang), Extrakte von Stengeln und Blättern (Geranium, Patchouli, Petitgrain), Extrakte von Früchten (Anis, Koriander, Kümmel, Wacholder), Extrakte von Fruchtschalen (Bergamotte, Zitrone, Orangen), Extrakte von Wurzeln (Macis, Angelica, Sellerie, Kardamon, Costus, Iris, Calmus), Extrakte von Hölzern (Pinien-, Sandel-, Guajak-, Zedern-, Rosenholz), Extrakte von Kräutern und Gräsern (Estragon, Zitronengras, Salbei, Thymian), Extrakte von Nadeln und Zweigen (Fichte, Tanne, Kiefer, Latschen), Extrakte von Harzen und Balsamen (Galbanum, Elemi, Benzoe, Myrrhe, Olibanum, Opoponax), Zibet, Castoreum, Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat, Citral, Citronellal, Citronellyloxyacetaldehyd, Cyclamenaldehyd, Hydroxycitronellal, Lilial, Bourgeonal, Isomethylionon, Methylcedrylketon, Anethol, Citronellol, Eugenol, Isoeugenol, Geraniol, Linalool, Phenylethylalkohol, Terpineol, Terpene, Balsame, Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl, Lavandinöl, Bergamotteöl, Dihydromyrcenol, Lyral, α-Hexylzimtaldehyd, Benzylaceton, Linalool, Ambroxan, Indol, Hedione, Sandelice, Citronenöl, Mandarinenöl, Orangenöl, Allylamylglycolat, Cyclovertal, ß-Damascone, Geraniumöl, Cyclohexylsalicylat, Phenylessigsäure, Geranylacetat und Rosenoxid.

Derartige Zusammensetzungen sind insbesondere deshalb bevorzugt, weil durch die ein, zwei oder mehr weiteren Bestandteile die physikalisch-chemischen und sensorischen Eigenschaften der Zusammensetzung gezielt gesteuert und den Anforderungen angepasst werden können, ohne den positiven Einfluss des in der vorstehend offenbarten Zusammensetzung enthaltenen N-Methyl-N-((2S,3R,4R,5R)-2,3,4,5,6-pentahydroxyhexyl)-(Z)-octadec-9-enamid auf die augenreizende Wirkung der anderen in der Zusammensetzung enthaltenen Tenside -und gegebenenfalls sonstiger augenreizender Stoffe- zu beeinträchtigen.

Insbesondere lassen sich durch die ein, zwei oder mehr weiteren Bestandteile die Viskosität, der Geruch, die Farbe und der pH-Wert einstellen sowie die Haltbarkeit der Zusammensetzung erhöhen. Insbesondere die Gegenwart von UV-Filtern, Konservierungsmitteln und/oder Antioxidantien ermöglicht es, die durch das N-Methyl-N-((2S,3R,4R,5R)-2,3,4,5,6-pentahydroxyhexyl)-(Z)-octadec-9-enamid erreichte Verringerung der augenreizenden Wirkung über einen langen Zeitraum zu erhalten.

Bevorzugt ist eine vorstehend offenbarte Zusammensetzung (vorzugsweise wie vorstehend als bevorzugt bezeichnet) mit einem pH-Wert im Bereich von 5,0 bis 8,0. Vorstehend offenbarte Zusammensetzungen mit einem pH in diesem Bereich werden als besonders augenmild empfunden. Der angegebene pH-Wert liegt hinreichend nah am pH-Wert von Tränen (ca. 7,35) und ist insbesondere deshalb bevorzugt, weil er ein günstiges Verhältnis von Reinigungsleistung und Augenmilde ermöglicht.

Bevorzugt ist auch eine vorstehend offenbarte Zusammensetzung (vorzugsweise wie vorstehend als bevorzugt bezeichnet) mit einer Viskosität im Bereich von 400 bis 7000 mPas, wobei die Viskosität mit einem Brookfield Rotationsviskosimeter mit einer LV2 Spindel, mit 4 UPM für den Bereich 4000-7000 mPas, mit 10 UPM für den Bereich 1800-4000, mit 12 UPM für den Bereich 1200-1800 mPas, mit 20 UPM für den Bereich 800-1200 mPas, mit 30 UPM für den Bereich von 600-800 mPas und mit 50 UPM im Bereich unter 600 mPas bei einer Temperatur von 20 °C und einem Druck von 1013 mbar bestimmt wird. Zusammensetzungen mit einer solchen Viskosität sind besonders gut als Handgeschirrspülmittel einsetzbar, wobei solche Handgeschirrspülmittel vorzugsweise in einer Quetschflasche bereitgestellt werden. Die angegebene Viskosität ist für Reinigungsmittel auch deshalb besonders bevorzugt, weil sie nach der Applikation die notwendige Verweilzeit auf einer zu entfernenden Verunreinigung z.B. auf Geschirr auch bei vertikaler Anordnung gewährleistet. Die Viskosität ist aber auch insbesondere deshalb bevorzugt, weil die Spritzneigung einer entsprechenden Zusammensetzung niedrig ist. Aufgrund ihrer besonderen Augenmilde bevorzugt ist auch eine vorstehend offenbarte Zusammensetzung (vorzugsweise wie vorstehend als bevorzugt bezeichnet) wobei die Zusammensetzung in einem "Red Blood Cell" (RBC)-Test gemäß W. Pape, U. Pfannenbecker, U. Hoppe; Molecular Toxicology, 1987, 1 (4), 525-536 als augenmilder bewertet wird als eine Vergleichszusammensetzung, die bei ansonsten gleichen Anteilen von Inhaltsstoffen keine N-Methyl-N-((2S,3R,4R,5R)-2,3,4,5,6-pentahydroxyhexyl)-Fettsäureamide enthält.

Bevorzugt ist eine vorstehend offenbarte Zusammensetzung (vorzugsweise wie vorstehend als bevorzugt bezeichnet) umfassend N-Methyl-N-((2S,3R,4R,5R)-2,3,4,5,6-pentahydroxyhexyl)-hexadecanamid, N-Methyl-N-((2S,3R,4R,5R)-2,3,4,5,6-pentahydroxyhexyl)-octadecanamid und N-Methyl-N-((2S,3R,4R,5R)-2,3,4,5,6-pentahydroxyhexyl)-(Z,Z)-octadeca-9, 12-dienamid,
wobei bevorzugt das Verhältnis der Gesamtmenge an N-Methyl-N-((2S,3R,4R,5R)-2,3,4,5,6-pentahydroxyhexyl)-hexadecanamid, N-Methyl-N-((2S,3R,4R,5R)-2,3,4,5,6-pentahydroxyhexyl)-octadecanamid und N-Methyl-N-((2S,3R,4R,5R)-2,3,4,5,6-pentahydroxyhexyl)-(Z,Z)-octadeca-9,12-dienamid zu der Gesamtmenge der Verbindung N-Methyl-N-((2S,3R,4R,5R)-2,3,4,5,6-pentahydroxyhexyl)-(Z)-octadec-9-enamid im Bereich von 0,08 bis 0,25 liegt.

Eine derartige Zusammensetzung ist deshalb bevorzugt, weil sie nicht nur sehr augenmild ist und dabei eine hohe Reinigungskraft besitzt, sondern insbesondere auch deshalb, weil die entsprechende Zusammensetzung sehr effizient und mit geringem Aufwand, insbesondere ohne die Notwendigkeit von vor- oder nachgelagerten Reinigungsschritten, ausgehend von regenerativen Rohstoffen hergestellt und verwendet werden kann. Bei ausschließlicher Verwendung von High Oleic Sonnenblumenöl als Fettsäurequelle für die Herstellung des N-Methyl-N-((2S,3R,4R,5R)-2,3,4,5,6-pentahydroxyhexyl)-(Z)-octadec-9-enamid bzw. bei alleiniger Verwendung von High Oleic Sonnenblumenöl als Umsetzungspartner für (2R,3R,4R,5S)-6-(Methylamino)hexan-1,2,3,4,5-pentol (bzw. ein zur entsprechenden Umsetzung befähigtes Derivat dieser Verbindung) entstehen regelmäßig die besagten, zusätzlich in der vorstehend offenbarten Zusammensetzung vorliegenden Verbindungen (Glucamide), üblicherweise in dem als bevorzugt angegebenen Mengenbereich. Soweit gewünscht, können Reinigungsschritte vorgesehen werden, um den angegebenen Mengenbereich einzustellen, nötig ist dies jedoch regelmäßig nicht.

Offenbart wird auch die Verwendung einer vorstehend offenbarten Zusammensetzung (vorzugsweise einer Zusammensetzung wie vorstehend als bevorzugt bezeichnet)
in einem bzw. als Spülmittel, vorzugsweise in einem bzw. als Handgeschirrspülmittel, oder
in einem bzw. als Reinigungsmittel für ein oder mehrere Substrate ausgewählt aus der Gruppe bestehend aus Haar, Haut, harte Oberflächen und Textilien, vorzugsweise Shampoo, Reinigungslotion, Flüssigseife, Duschgel oder Badezusatz.

Bedingt durch die hohe Reinigungskraft der vorstehend offenbarten Zusammensetzungen können diese als Spülmittel oder Reinigungsmittel verwendet werden. Da die vorstehend offenbarten Zusammensetzungen besonders augenmild sind (wegen der Anwesenheit von N-Methyl-N-((2S,3R,4R,5R)-2,3,4,5,6-pentahydroxyhexyl)-(Z)-octadec-9-enamid), ist die Verwendung in solchen Bereichen vorteilhaft, in denen die Wahrscheinlichkeit eines Augenkontaktes besonders groß ist. Aus diesem Grund ist die Verwendung als Handgeschirrspülmittel oder im Bereich der Körperpflege bevorzugt.

Besonders vorteilhaft ist die offenbarte Verwendung, da das in vorstehend offenbarten Zusammensetzungen enthaltene N-Methyl-N-((2S,3R,4R,5R)-2,3,4,5,6-pentahydroxyhexyl)-(Z)-octadec-9-enamid in der Natur und in Kläranlagen leicht abbaubar ist.

Eine Vielzahl der vorstehend genannten Aufgaben wird gelöst durch die Verwendung von N-Methyl-N-((2S,3R,4R,5R)-2,3,4,5,6-pentahydroxyhexyl)-(Z)-octadec-9-enamid oder einer Mischung umfassend N-Methyl-N-((2S,3R,4R,5R)-2,3,4,5,6-pentahydroxyhexyl)-(Z)-octadec-9-enamid sowie ein, zwei oder mehr weitere N-Methyl-N-((2S,3R,4R,5R)-2,3,4,5,6-pentahydroxyhexyl)-Fettsäureamide, als Additiv zur Verringerung der augenreizenden Wirkung
- von Spülmitteln umfassend ein, zwei oder mehr Tenside ausgewählt aus der Gruppe bestehend aus Sodium Laureth Sulfate, Cocoamidopropyl Betain und Sodium Laurylsulfate, vorzugsweise Handgeschirrspülmitteln,
   oder
- von Reinigungsmitteln umfassend ein, zwei oder mehr Tenside ausgewählt aus der Gruppe bestehend aus Sodium Laureth Sulfate, Cocoamidopropyl Betain und Sodium Laurylsulfate, für ein oder mehrere Substrate ausgewählt aus der Gruppe bestehend aus Haar, Haut, harte Oberflächen und Textilien, vorzugsweise Shampoo, Reinigungslotion, Flüssigseife, Duschgel oder Badezusatz.

Hierbei ist vorzugsweise das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide in der Mischung größer als 0,75. Hinsichtlich der bevorzugten Anteile an Tensiden und sonstigen Bestandteilen in den Spülmitteln bzw. Reinigungsmitteln sei auf die obigen Ausführungen zu vorstehend offenbarten Zusammensetzungen verwiesen, die analog zutreffen, *mutatis mutandis.*

Überraschenderweise wurde gefunden, dass N-Methyl-N-((2S,3R,4R,5R)-2,3,4,5,6-pentahydroxyhexyl)-(Z)-octadec-9-enamid bei Einsatz als Additiv in Spülmitteln oder Reinigungsmitteln die augenreizende Wirkung dieser Mittel verringert. Dass die augenreizende Wirkung der in Spül- und Reinigungsmitteln enthaltenen Tenside durch die Zugabe einer Verbindung, die selbst als Tensid wirksam ist, verringert werden kann, ist für den Fachmann völlig unerwartet.

Durch die hohe Waschleistung und die inhärente Augenmilde von N-Methyl-N-((2S,3R,4R,5R)-2,3,4,5,6-pentahydroxyhexyl)-(Z)-octadec-9-enamid, sowie die Tatsache, dass es in der Natur und in Kläranlagen leicht abbaubar ist, ist die Verwendung vorstehend offenbarter Zusammensetzungen als Additiv in solchen Produkten bevorzugt, für die die Wahrscheinlichkeiten eines Augenkontaktes oder der Freisetzung in die Umwelt besonders groß sind, wie Handgeschirrspülmittel oder Reinigungsmitteln für den Körper (Körperpflegeprodukte).

Eine Vielzahl der vorstehend genannten Aufgaben werden gelöst durch die Verwendung von N-Methyl-N-((2S,3R,4R,5R)-2,3,4,5,6-pentahydroxyhexyl)-(Z)-octadec-9-enamid oder einer Mischung umfassend N-Methyl-N-((2S,3R,4R,5R)-2,3,4,5,6-pentahydroxyhexyl)-(Z)-octadec-9-enamid sowie ein, zwei oder mehr weitere N-Methyl-N-((2S,3R,4R,5R)-2,3,4,5,6-pentahydroxyhexyl)-Fettsäureamide, als Mittel zur Verringerung der augenreizenden Wirkung von ein, zwei oder mehr Tensiden ausgewählt aus der Gruppe bestehend aus Sodium Laureth Sulfate, Cocoamidopropyl Betain und Sodium Laurylsulfate, sowie vorzugsweise zusätzlich zur Verringerung der augenreizenden Wirkung von Lauryl/Myristyl Glucoside und/oder PEG-4 Rapeseedamide, bevorzugt in einem Spülmittel, vorzugsweise Handgeschirrspülmittel, oder in einem Reinigungsmittel für ein oder mehrere Substrate ausgewählt aus der Gruppe bestehend aus Haar, Haut, harte Oberflächen und Textilien, vorzugsweise von Shampoo, Reinigungslotion, Flüssigseife, Duschgel oder Badezusatz.

Hierbei ist vorzugsweise das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide in der Mischung größer als 0,75. Hinsichtlich der bevorzugten Anteile an Tensiden und sonstigen Bestandteilen in den Spülmitteln bzw. Reinigungsmitteln sei auf die obigen Ausführungen zu vorstehend offenbarten Zusammensetzungen verwiesen, die analog zutreffen, *mutatis mutandis.*

Die vorstehend angeführten Vorteile sind besonders ausgeprägt für Spülmittel oder Reinigungsmittel, die zumindest ein Tensid ausgewählt aus der Gruppe bestehend aus Lauryl/Myristyl Glucoside und PEG-4 Rapeseedamide umfassen.

Die vorliegende Erfindung betrifft demnach die Verwendung von N-Methyl-N-((2S,3R,4R,5R)-2,3,4,5,6-pentahydroxyhexyl)-(Z)-octadec-9-enamid oder einer Mischung umfassend N-Methyl-N-((2S,3R,4R,5R)-2,3,4,5,6-pentahydroxyhexyl)-(Z)-octadec-9-enamid sowie ein, zwei oder mehr weitere N-Methyl-N-((2S,3R,4R,5R)-2,3,4,5,6-pentahydroxyhexyl)-Fettsäureamide,
- als Additiv zur Verringerung der augenreizenden Wirkung von Spülmitteln umfassend ein, zwei oder mehr Tenside ausgewählt aus der Gruppe bestehend aus Sodium Laureth Sulfate, Cocoamidopropyl Betain und Sodium Laurylsulfate oder von Reinigungsmitteln umfassend ein, zwei oder mehr Tenside ausgewählt aus der Gruppe bestehend aus Sodium Laureth Sulfate, Cocoamidopropyl Betain und Sodium Laurylsulfate, für ein oder mehrere Substrate ausgewählt aus der Gruppe bestehend aus Haar, Haut, harte Oberflächen und Textilien
   oder
- als Mittel zur Verringerung der augenreizenden Wirkung von ein, zwei oder mehr Tensiden ausgewählt aus der Gruppe bestehend aus Sodium Laureth Sulfate, Cocoamidopropyl Betain und Sodium Laurylsulfate.

Bevorzugt ist eine erfindungsgemäße Verwendung, wobei das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide in der Mischung größer als 0,75 ist.

Bevorzugt ist auch eine erfindungsgemäße Verwendung (vorzugsweise wie vorstehend als bevorzugt bezeichnet),
- als Additiv zur Verringerung der augenreizenden Wirkung von Handgeschirrspülmitteln umfassend ein, zwei oder mehr Tenside ausgewählt aus der Gruppe bestehend aus Sodium Laureth Sulfate, Cocoamidopropyl Betain und Sodium Laurylsulfate oder von Shampoos, Reinigungslotionen, Flüssigseifen, Duschgelen oder Badezusätzen umfassend ein, zwei oder mehr Tenside ausgewählt aus der Gruppe bestehend aus Sodium Laureth Sulfate, Cocoamidopropyl Betain und Sodium Laurylsulfate, für ein oder mehrere Substrate ausgewählt aus der Gruppe bestehend aus Haar, Haut, harte Oberflächen und Textilien
   oder
- als Mittel zur Verringerung der augenreizenden Wirkung von ein, zwei oder mehr Tensiden ausgewählt aus der Gruppe bestehend aus Sodium Laureth Sulfate, Cocoamidopropyl Betain und Sodium Laurylsulfate, sowie vorzugsweise zusätzlich zur Verringerung der augenreizenden Wirkung von Lauryl/Myristyl Glucoside und/oder PEG-4 Rapeseedamide, in einem Handgeschirrspülmittel oder in einem Reinigungsmittel für ein oder mehrere Substrate ausgewählt aus der Gruppe bestehend aus Haar, Haut, harte Oberflächen und Textilien, vorzugsweise von Shampoo, Reinigungslotion, Flüssigseife, Duschgel oder Badezusatz.

Offenbart wird auch ein Verfahren zur Herstellung einer vorstehend offenbarten Zusammensetzung (vorzugsweise wie vorstehend als bevorzugt bezeichnet), mit folgenden Schritten:
a) Bereitstellen oder Herstellen eines Startmaterials ausgewählt aus der Gruppe bestehend aus
   High Oleic Sonnenblumenöl,
   Mischung von Fettsäuren aus High Oleic Sonnenblumenöl und/oder deren Salzen, Mischung von Estern von Fettsäuren aus High Oleic Sonnenblumenöl,
   Mischung von Fettsäurechloriden von Fettsäuren aus High Oleic Sonnenblumenöl und deren Mischungen,
b) Umsetzen des Startmaterials mit (2R,3R,4R,5S)-6-(Methylamino)hexan-1,2,3,4,5-pentol.

Aus Schritt b) resultiert -entsprechend dem Fettsäuremuster des High Oleic Sonnenblumenöls- eine Mischung umfassend N-Methyl-N-((2S,3R,4R,5R)-2,3,4,5,6-pentahydroxyhexyl)-(Z)-octadec-9-enamid sowie weitere N-Methyl-N-((2S,3R,4R,5R)-2,3,4,5,6-pentahydroxyhexyl)-Fettsäureamide, wobei das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide größer als 0,75 ist. Zum Fettsäuremuster (der Fettsäurezusammensetzung) eines typischen High Oleic Sonnenblumenöls (= ölsäurereiches Sonnenblumenöl) im Vergleich mit anderen Ölen vergleiche Bundesministerium für Ernährung, Landwirtschaft und Verbraucherschutz: Deutsches Lebensmittelbuch - Leitsätze für Speisefette und Speiseöle, 2008.

Die aus Schritt b) resultierende Mischung (in Ausnahmefällen: das eingesetzte Startmaterial) wird dann, gegebenenfalls nach weiteren Behandlungsschritten, regelmäßig mit weiteren (vorzugsweise sämtlichen weiteren) Bestandteilen der herzustellenden vorstehend offenbarten Zusammensetzung vermischt. Lediglich optional werden die aus Schritt b) resultierende Mischung oder eine daraus durch Vermischen resultierende (Folge- oder Produkt-)Mischung aufgereinigt, erhitzt oder auf andere Weise behandelt.

Das vorstehend offenbarte Verfahren ist besonders vorteilhaft, weil die Herstellung einer vorstehend offenbarten Zusammensetzung enthaltend N-Methyl-N-((2S,3R,4R,5R)-2,3,4,5,6-pentahydroxyhexyl)-(Z)-octadec-9-enamid aus dem natürlichen Rohstoff High Oleic Sonnenblumenöl erfolgt, der in den natürlichen CO₂-Kreislauf eingebunden ist. Darüber hinaus basiert (2R,3R,4R,5S)-6-(Methylamino)hexan-1,2,3,4,5-pentol auf dem natürlichen Rohstoff D-Glucose, der ebenfalls in den natürlichen CO₂-Kreislauf eingebunden ist. Besonders vorteilhaft ist das vorstehend offenbarte Verfahren auch deshalb, weil es mit einer hohen Effizienz durchgeführt werden kann und keine vor- oder nachgelagerten Reinigungsschritte erfordert. Darüber hinaus ist besonders vorteilhaft, dass als wesentliches Nebenprodukt des Verfahrens lediglich Wasser anfällt, so dass keine unerwünschten Abfälle entstehen.

Der zusätzliche Einsatz weiterer Glucamide auf Basis anderer Pflanzenöle, entsprechender Fettsäuremischungen, Fettsäureestermischungen oder Fettsäurechloridmischungen ist im Rahmen der vorliegenden Erfindung, d.h. zur Lösung der zugrundeliegenden technischen Aufgabe, nicht erforderlich und regelmäßig schon aus Gründen der Nachhaltigkeit nicht erwünscht.

Ein bevorzugtes vorstehend offenbartes Verfahren zur Herstellung einer vorstehend offenbarten Zusammensetzung umfasst daher nicht den Schritt des Umsetzens von (2R,3R,4R,5S)-6-(Methylamino)hexan-1,2,3,4,5-pentol mit einem anderen Pflanzenöl, einer entsprechenden anderen Fettsäuremischung, einer entsprechenden anderen Fettsäureestermischung oder einer entsprechenden anderen Fettsäurechloridmischung (mit somit anderem Fettsäuremuster).

Es ist somit bevorzugt, wenn in einer vorstehend offenbarten Zusammensetzung das Fettsäuremuster der N-Methyl-N-((2S,3R,4R,5R)-2,3,4,5,6-pentahydroxyhexyl)-Fettsäureamide mit dem Fettsäuremuster des im vorstehend offenbarten Herstellverfahren eingesetzten Startmaterials (wie oben definiert) übereinstimmt. Das Fettsäuremuster der vorstehend offenbarten Zusammensetzung ist dann ein Zertifikat für den Einsatz der nachhaltigen Rohstoffquelle High Oleic Sonnenblumenöl und für die Anbauregion der zugrundeliegenden High Oleic Sonnenblume.

Offenbart wird auch ein Verfahren zur Herstellung eines Spülmittels, vorzugsweise Handgeschirrspülmittels, oder eines Reinigungsmittels für ein oder mehrere Substrate ausgewählt aus der Gruppe bestehend aus Haar, Haut, harte Oberflächen und Textilien, vorzugsweise von Shampoo, Reinigungslotion, Flüssigseife, Duschgel oder Badezusatz mit folgenden Schritten:
- Herstellen gemäß dem vorstehend offenbarten Verfahren oder Bereitstellen einer vorstehend offenbarten Zusammensetzung (vorzugsweise wie vorstehend als bevorzugt bezeichnet),
- Mischen des hergestellten oder bereitgestellten Zusammensetzung mit ein oder mehr weiteren Tensiden sowie weiteren Bestandteilen.

Mit diesem vorstehend offenbarten Verfahren können Spülmittel oder Reinigungsmittel für ein oder mehrere Substrate ausgewählt aus der Gruppe bestehend aus Haar, Haut, harte Oberflächen und Textilien hergestellt werden, die über eine ausgezeichnete Reinigungskraft verfügen und dabei besonders augenmild sind.

Die Erfindung wird nachfolgend anhand von Beispielen näher erläutert.

### Beispiele:

Da die Reinigungskraft sowie die haut- und augenreizende Wirkung von Spül- und Reinigungsmitteln, insbesondere Handgeschirrspülmitteln, Shampoos, Reinigungslotionen, Flüssigseifen, Duschgelen oder Badezusätzen maßgeblich durch die enthaltenen Tenside bestimmt wird, erfolgt der Vergleich der vorstehend offenbarten Zusammensetzungen nachfolgend jeweils mit wasserbasierten Tensid-Zusammensetzungen (und in Abwesenheit ansonsten üblicher Additive oder Bestandteile wie pH-Regulatoren, Komplexiermitteln, Salzen, rheologischen Additiven, Enzymen, Vitaminen, Konservierungsmittel, UV-Filtern, Antioxidantien, Trübungsmitteln, Farbstoffen, Riechstoffen oder weiteren Lösungsmitteln). Soweit nicht anders angegeben, sind sämtliche Zusammensetzungen in Gew.-% angegeben.

In vorstehend offenbarten Zusammensetzungen und in Vergleichszusammensetzungen werden in den Beispielen neben N-Methyl-N-((2S,3R,4R,5R)-2,3,4,5,6-pentahydroxyhexyl)-Fettsäureamiden und dem noch recht selten in handelsüblichen Zusammensetzungen eingesetzten, augenreizenden Tensid PEG-4 Rapeseedamide insbesondere Tenside eingesetzt, die ausgewählt sind aus der Gruppe bestehend aus Sodium Laureth Sulfate, Cocoamidopropyl Betain, Lauryl/Myristyl Glucoside und Sodium Laurylsulfate. Diese Tenside sind übliche, augenreizende Bestandteile von Spülmitteln und Reinigungsmitteln.

### Reinigungskraft:

Die Reinigungskraft wurde mit Hilfe eines Teller-Tests (IKW-Schmutzart 2; vgl. "Recommendation for the quality assessment of the cleaning performance of hand dishwashing detergents"; C. Nitsch, G. Hüttmann; SÖFW-Journal, 128, 5, 11-15, 2002) ermittelt. Es wurden pro Liter Spüllösung jeweils 1 mL der zu untersuchenden Probe eingesetzt.

Die Zusammensetzungen der untersuchten Proben sowie die Ergebnisse des Teller-Tests sind Tabelle 1 zusammengestellt.

### Augenmilde:

Die augenreizende Wirkung von zu untersuchenden Proben wurde mit Hilfe des "Red Blood Cell" Tests (RBC; gemäß W. Pape, U. Pfannenbecker, U. Hoppe; "Validation of the red blood cell test as an in vitro assay for the rapid screening of irritation potential of surfactants", Molecular Toxicology, 1987, 1 (4), 525-536) ermittelt.

Die Zusammensetzungen der untersuchten Proben sind in Tabelle 2 zusammengestellt.

Die Ergebnisse des RBC-Test sind in Tabelle 3 zusammengestellt, in der die Augenmilde jeweils im direkten Vergleich zwischen zwei Proben bewertet wird. Die im RBC-Test als augenmilder gefundene Probe wird hierbei durch ein "+"und die weniger augenmilde entsprechend durch ein "-" gekennzeichnet.

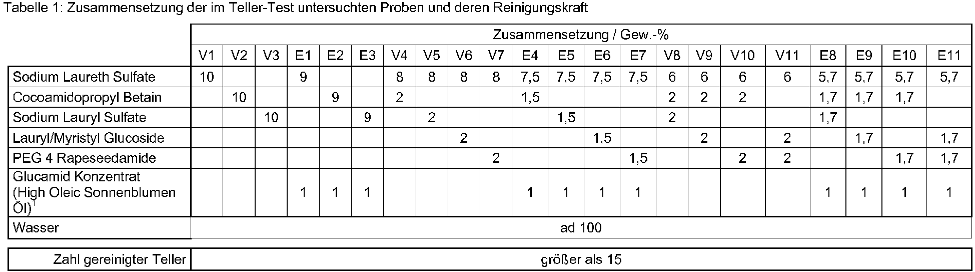

**Tabelle 2: Zusammensetzung der im RBC-Test untersuchten Proben**

| | Zusammensetzung / Gew.-% | | | | | | | | | | |
|---|---|---|---|---|---|---|---|---|---|---|---|
| | V12a | V12b | V13 | V14 | E12 | E13 | E14 | V15 | E15 | V16 | E16 |
| Sodium Laureth Sulfate | 10 | 10 | | | 10 | | | 6 | 6 | 6 | 6 |
| Cocoamidopropyl Betain | | | 10 | | | 10 | | 1 | 1 | 1 | 1 |
| Sodium Lauryl Sulfate | | | | 10 | | | 10 | 1 | 1 | 1 | 1 |
| Lauryl/Myristyl Glucoside | | | | | | | | | | 1 | 1 |
| PEG 4 Rapeseedamide | | | | | | | | 2 | 2 | 1 | 1 |
| Glucamid Konzentrat (Kokosöl)2 | | 1 | | | | | | | | | |
| Glucamid Konzentrat (High Oleic Sonnenblumen Öl) | | | | | 1 | 1 | 1 | | 1 | | 1 |
| Wasser | ad 100 | | | | | | | | | | |

| | | | | | | | | | | | |
|---|---|---|---|---|---|---|---|---|---|---|---|
| ¹: Glucamid Konzentrat (High Oleic Sonnenblumenöl) bezeichnet ein Konzentrat aus N-Methylglucamiden, welches das Umsetzungsprodukt der Umsetzung von High Oleic Sonnenblumenöl mit (2R,3R,4R,5S)-6-(Methylamino)hexan-1,2,3,4,5-pentol enthält und das mehr als 75 Gew.-% N-Methyl-N-((2S,3R,4R,5R)-2,3,4,5,6-pentahydroxyhexan)-(Z)-octadec-9-enamid enthält. ²: Glucamid Konzentrat (Kokosöl) bezeichnet ein Konzentrat aus N-Methylglucamiden, welches das Umsetzungsprodukt der Umsetzung von Kokosöl mit (2R,3R,4R,5S)-6-(Methylamino)hexan-1,2,3,4,5-pentol enthält und das weniger als 10 Gew.-% N-Methyl-N-((2S,3R,4R,5R)-2,3,4,5,6-pentahydroxyhexan)-(Z)-octadec-9-enamid enthält. Das Glucamid Konzentrat (Kokosöl) besteht zu mehr als 62 Gew.-% aus N-Methyl-N-((2S,3R,4R,5R)-2,3,4,5,6-pentahydroxyhexan)-dodecanamid und N-Methyl-N-((2S,3R,4R,5R)-2,3,4,5,6-pentahydroxyhexan)-tetradecanamid. | | | | | | | | | | | |

**Tabelle 3: Bewertung der Augenmilde anhand der Ergebnisse des RBC-Test im direkten Vergleich zwischen Beispielen (zu vorstehend offenbarten Zusammensetzungen) und Vergleichsbeispielen. Die im RBC-Test als augenmilder gefundene Probe wird durch ein unmittelbar benachbartes "+" und die weniger augenmilde entsprechend durch ein "-" gekennzeichnet.**

| Vergleich | Vergleich der Augenmilde | | Beispiel |
|---|---|---|---|
| V12a | - | + | E12 |
| V12b | - | + | E12 |
| V13 | - | + | E13 |
| V14 | - | + | E14 |
| V15 | - | + | E15 |
| V16 | - | + | E16 |

## Patentansprüche

1. Verwendung von N-Methyl-N-((2S,3R,4R,5R)-2,3,4,5,6-pentahydroxyhexyl)-(Z)-octadec-9-enamid oder einer Mischung umfassend N-Methyl-N-((2S,3R,4R,5R)-2,3,4,5,6-pentahydroxyhexyl)-(Z)-octadec-9-enamid sowie ein, zwei oder mehr weitere N-Methyl-N-((2S,3R,4R,5R)-2,3,4,5,6-pentahydroxyhexyl)-Fettsäureamide,
- als Additiv zur Verringerung der augenreizenden Wirkung von Spülmitteln umfassend ein, zwei oder mehr Tenside ausgewählt aus der Gruppe bestehend aus Sodium Laureth Sulfate, Cocoamidopropyl Betain und Sodium Laurylsulfate oder von Reinigungsmitteln umfassend ein, zwei oder mehr Tenside ausgewählt aus der Gruppe bestehend aus Sodium Laureth Sulfate, Cocoamidopropyl Betain und Sodium Laurylsulfate, für ein oder mehrere Substrate ausgewählt aus der Gruppe bestehend aus Haar, Haut, harte Oberflächen und Textilien
oder
- als Mittel zur Verringerung der augenreizenden Wirkung von ein, zwei oder mehr Tensiden ausgewählt aus der Gruppe bestehend aus Sodium Laureth Sulfate, Cocoamidopropyl Betain und Sodium Laurylsulfate.

2. Verwendung nach Anspruch 1, wobei das Verhältnis der Masse von N-Methyl-N-((2S,3R,4R,5R)-2,3,4,5,6-pentahydroxyhexyl)-(Z)-octadec-9-enamid zur Gesamtmasse sämtlicher N-Methyl-N-((2S,3R,4R,5R)-2,3,4,5,6-pentahydroxyhexyl)-Fettsäureamide in der Mischung größer als 0,75 ist.

3. Verwendung nach einem der Ansprüche 1 oder 2,
- als Additiv zur Verringerung der augenreizenden Wirkung von Handgeschirrspülmitteln umfassend ein, zwei oder mehr Tenside ausgewählt aus der Gruppe bestehend aus Sodium Laureth Sulfate, Cocoamidopropyl Betain und Sodium Laurylsulfate oder von Shampoos, Reinigungslotionen, Flüssigseifen, Duschgelen oder Badezusätzen umfassend ein, zwei oder mehr Tenside ausgewählt aus der Gruppe bestehend aus Sodium Laureth Sulfate, Cocoamidopropyl Betain und Sodium Laurylsulfate, für ein oder mehrere Substrate ausgewählt aus der Gruppe bestehend aus Haar, Haut, harte Oberflächen und Textilien
oder
- als Mittel zur Verringerung der augenreizenden Wirkung von ein, zwei oder mehr Tensiden ausgewählt aus der Gruppe bestehend aus Sodium Laureth Sulfate, Cocoamidopropyl Betain und Sodium Laurylsulfate, sowie vorzugsweise zusätzlich zur Verringerung der augenreizenden Wirkung von Lauryl/Myristyl Glucoside und/oder PEG-4 Rapeseedamide, in einem Handgeschirrspülmittel oder in einem Reinigungsmittel für ein oder mehrere Substrate ausgewählt aus der Gruppe bestehend aus Haar, Haut, harte Oberflächen und Textilien, vorzugsweise von Shampoo, Reinigungslotion, Flüssigseife, Duschgel oder Badezusatz.

## Claims

1. Use of N-Methyl-N-((2S,3R,4R,5R)-2,3,4,5,6-pentahydroxyhexyl)-(Z)-octadec-9-enamide or a mixture comprising N-Methyl-N-((2S,3R,4R,5R)-2,3,4,5,6-pentahydroxyhexyl)-(Z)-octadec-9-enamide and one, two or more additional N-Methyl-N-((2S,3R,4R,5R)-2,3,4,5,6-pentahydroxyhexyl) fatty acid amides,
- as an additive to reduce the eye-irritating effect of detergents comprising one, two or more surfactants selected from the group consisting of sodium laureth sulphate, cocamidopropyl betaine and sodium lauryl sulphate or of cleaning agents comprising one, two or more surfactants selected from the group consisting of sodium laureth sulphate, cocamidopropyl betaine and sodium lauryl sulphate for one or a plurality of substrates selected from the group consisting of hair, skin, hard surfaces and textiles
or
- as an agent to reduce the eye-irritating effect of one, two or more surfactants selected from the group consisting of sodium laureth sulphate, cocamidopropyl betaine and sodium lauryl sulphate.

2. Use according to claim 1, wherein the ratio of the mass of N-Methyl-N-((2S,3R,4R,5R)-2,3,4,5,6-pentahydroxyhexyl)-(Z)-octadec-9-enamide to the total mass of all N-Methyl-N-((2S,3R,4R,5R)-2,3,4,5,6-pentahydroxyhexyl) fatty acid amides in the mixture is greater than 0.75.

3. Use according to any one of claims 1 or 2,
- as an additive to reduce the eye-irritating effect of dishwasher detergents comprising one, two or more surfactants selected from the group consisting of sodium laureth sulphate, cocamidopropyl betaine and sodium lauryl sulphate or of shampoos, cleansing lotions, liquid soaps, shower gels or bath additives comprising one, two or more surfactants selected from the group consisting of sodium laureth sulphate, cocamidopropyl betaine and sodium lauryl sulphate for one or a plurality of substrates selected from the group consisting of hair, skin, hard surfaces and textiles
or
- as an agent to reduce the eye-irritating effect of one, two or more surfactants selected from the group consisting of sodium laureth sulphate, cocamidopropyl betaine and sodium lauryl sulphate and preferably also to reduce the eye-irritating effect of lauryl/myristyl glucosides and/or PEG-4 rapeseed amides in a dishwasher detergent or in a cleansing agent for one or a plurality of substrates selected from the group consisting of hair, skin, hard surfaces and textiles, preferably of shampoo, cleansing lotion, liquid soap, shower gel or bath additive.

## Revendications

1. Utilisation de N-méthyl-N-((2S,3R,4R,5R)-2,3,4,5,6-pentahydroxyhexyl)-(Z)-octadec-9-enamide ou d'un mélange comprenant du N-méthyl-N-((2S,3R,4R,5R)-2,3,4,5,6-pentahydroxyhexyl)-(Z)-octadec-9-enamide, ainsi qu'un, deux ou plusieurs autres amides d'acide gras à base de N-méthyl-N-((2S,3R,4R,5R)-2,3,4,5,6-pentahydroxyhexyle),
- comme additif servant à réduire l'effet irritant pour les yeux de produits de rinçage comprenant un, deux ou plusieurs tensioactifs sélectionnés parmi le groupe constitué de sodium laureth sulfate, de cocoamidopropyle bétaïne et de sodium lauryl sulfate ou de produits de nettoyage comprenant un, deux ou plusieurs tensioactifs sélectionnés parmi le groupe constitué de sodium laureth sulfate, de cocoamidopropyle betaïne et de sodium lauryl sulfate, pour un ou plusieurs substrats sélectionnés parmi le groupe constitué de cheveux, de peau, de surfaces dures et de textiles,
ou
- comme agent servant à réduire l'effet irritant pour les yeux d'un, de deux ou de plusieurs tensioactifs sélectionnés parmi le groupe constitué de sodium laureth sulfate, de cocoamidopropyle bétaïne et de sodium laurylsulfate.

2. Utilisation suivant la revendication 1, le rapport de la masse de N-méthyl-N-((2S,3R,4R,5R)-2,3,4,5,6-pentahydroxyhexyl)-(Z)-octadec-9-enamide à la masse totale de l'ensemble des amides d'acide gras N-méthyl-N-((2S,3R,4R,5R)-2,3,4,5,6-pentahydroxyhexyle) en mélange étant supérieur à 0,75.

3. Utilisation suivant une des revendications 1 ou 2,
- Comme additif pour réduire l'effet irritant pour les yeux de détergents pour vaisselle à main comprenant un, deux ou plusieurs tensioactifs sélectionnés parmi le groupe constitué de sodium laureth sulfate, de cocoamidopropyle bétaïne et de sodium lauryl sulfate ou de shampoings, de lotions nettoyantes, de savons liquides, de gels douche ou d'additifs de bain comprenant un, deux ou plusieurs tensioactifs sélectionnés parmi le groupe constitué de sodium laureth sulfate, de cocoamidopropyle bétaïne et de sodium lauryl sulfate pour un ou plusieurs substrats sélectionnés parmi le groupe constitué de cheveux, peau, surfaces dures et textiles
ou
- comme agent servant à réduire l'effet irritant pour les yeux d'un, de deux ou de plusieurs tensioactifs sélectionnés parmi le groupe constitué de sodium laureth sulfates, de cocoamidopropyle bétaïne et de sodium lauryl sulfate, ainsi que, de préférence, en plus, à réduire l'effet irritant pour les yeux de glucosides de lauryle/myristyle et/ou de PEG-4 rapseedamides, dans un détergent pour vaisselle à main ou dans un produit de nettoyage pour un ou plusieurs substrats sélectionnés parmi le groupe constitué de cheveux, de peau, de surfaces dures et textiles, de préférence de shampoings, de lotions nettoyantes, de savons liquides, de gels douche ou d'additifs de bain.
